# EUROPEAN PATENT SPECIFICATION

(11) **EP 1 543 838 B1**
(45) Date of publication and mention of the grant of the patent: **15.09.2010**
(21) Application number: 03738684.4
(22) Date of filing: 04.07.2003
(51) Int. Cl.: A61K 39/215, A61K 39/395, A61K 48/00, A61P 31/12, C12Q 1/68, G01N 33/569, C12N 15/09

(54) **FELINE INFECTIOUS PERITONITIS VACCINE**
VAKZINE GEGEN INFEKTIÖSE KATZEN-PERITONITIS
VACCIN CONTRE LA PERITONITE INFECTIEUSE FELINE

(30) Priority: 04.07.2002 JP 2002196290
(43) Date of publication of application: 22.06.2005
(73) Proprietor: The Kitasato Institute (formerly known as School Juridical Person Kitasato Gakuen), Minato-ku Tokyo 108-8641 (JP)
(72) Inventor: MOTOKAWA, Kenji, Kitamoto-shi, Saitama 364-0026 (JP); KUSUHARA, Hajime, Kitamoto-shi, Saitama 364-0026 (JP); KOYAMA, Hiroyuki, Towada-shi, Aomori 034-8628 (JP); HOHDATSU, Tsutomu, Towada-shi, Aomori 034-8628 (JP); ARAI, Setsuo, Kitamoto-shi, Saitama 364-0026 (JP)
(74) Representative: Grünecker, Kinkeldey, Stockmair & Schwanhäusser Anwaltssozietät
(86) International application number: PCT/JP2003/008524
(87) International publication number: WO 2004/004760

(56) References cited:
- EP-A1- 0 376 744
- EP-A1- 0 411 684
- EP-A2- 0 652 287
- WO-A1-95/08575
- WO-A1-97/20054
- WO-A2-02/72763
- WO-A2-02/92827
- JP-A- 2000 302 692
- US-B1- 6 241 989
- CORAPI W V ET AL: "MONOCLONAL ANTIBODY ANALYSIS OF NEUTRALIZATION AND ANTIBODY-DEPENDENT ENHANCEMENT OF FELINE INFECTIOUS PERITONITIS VIRUS" JOURNAL OF VIROLOGY, NEW YORK, US, US, vol. 66, no. 11, November 1992 (1992-11), pages 6695-6705, XP008022397 ISSN: 0022-538X
- GLANSBEEK HARRIE L ET AL: "Adverse effects of feline IL-12 during DNA vaccination against feline infectious peritonitis virus" JOURNAL OF GENERAL VIROLOGY, vol. 83, no. 1, January 2002 (2002-01), pages 1-10, XP009090038 ISSN: 0022-1317
- MOTOKAWA K. ET AL.: 'Comparison of the amino acid sequence and phylogenetic analysis of the promoter, integral membrane and nucleocapsid proteins of feline, canine and porcine coronaviruses' MICROBIOL. IMMUNOL. vol. 40, no. 6, 1996, pages 425 - 433, XP002109383
- VENNEMA H. ET AL.: 'Primary structure of the membrane and nucleocapsid protein genes of feline infectious peritonitis virus and immunogenicity of recombinant vaccinia viruses in kittens' VIROLOGY vol. 181, no. 1, 1991, pages 327 - 335, XP000604544
- DATABASE MEDLINE [Online] NLM8830483 WASMOEN T.L. ET AL.: 'Protection of cats from infectious peritonitis by vaccination with a recombinant raccoon poxvirus expressing nucleocapsid gene of feline infectious peritonitis virus', XP003019045 Retrieved from STN Database accession no. 96112252 & ADV. EXP. MED. BIOL. vol. 380, 1995, pages 221 - 228

## Description

### Technical Field

The present invention relates to the prevention or treatment of feline infectious peritonitis (FIP), which is caused by infection with feline infectious peritonitis virus (FIPV).

### Background Art

FIP is a complicated disease involving viral infection and the immune mechanism. FIP is a chronic and progressive disease characterized by pyogenic granulomas in the abdominal cavity, and ascites accumulation. Pyogenic granulomas form small gray-white plaques on all the surfaces of the abdominal cavities. These lesions are not limited to the inside of the abdominal cavity, and may be located in any organ over the entire body. The types of FIP can be categorized clinically and pathologically into the ascites type (effusive type), and the dry type (noneffusive type). The former is characterized by fibrinous peritonitis and the resulting accumulation of ascites. The latter is characterized by multiple pyogenic granuloma formation in various organs. However, the two types are not completely independent of each other, and cases in which both coexist are not rare. The type of disease that will develop after infection is considered to be determined by the strength of the infected cat's cellular immunity.

FIPV is an enveloped virus of approximately 100 to 150 nm in diameter, and belongs to the coronavirus genus in the Coronavirus family. The envelope surface has spikes approximately 20 nm long with enlarged tips, and can be said to resemble a crown. The viral genome comprises one molecule of single-stranded RNA. The virus replicates in the cytoplasm, buds through the endoplasmic reticulum, and matures. The virus is composed of the following three major structural proteins:
nucleocaspid (N) protein;
transmembrane (M) protein; and
peplomer (S) protein.

FIPV can be classified into type I, which proliferates slowly, and type II, which proliferates quickly. The nucleotide sequences of the genes of some FIPVs classified as type I are reported to vary, however, there are no such reports for FIPVs classified as type II. Meanwhile, approximately 70% of FIPV infections in cats are reported to be due to type I viruses (Hohdatsu, et al., Arch. Virol. 117, 85, 1991).

Many infection experiments and virological and immunological studies have been carried out regarding the mechanism of FIP pathogenesis. As a result, the immune system has been found to be involved in worsening FIPV infection symptoms.

Macrophages generally play an important role as one of the non-specific biophylaxis factors against viral infection. On the other hand, the biophylaxis mechanism of macrophages has sometimes been found to enhance infection. For example, a phenomenon whereby antibodies enhance infection has been confirmed for several viral infections, including FIP. More specifically, it has been reported that viruses bound to specific antibodies enter macrophages through Fc receptors, resulting in promoted infection and exacerbated symptoms. Viral entry into macrophages, mediated by antibodies and Fc receptors, does not require viral receptors on the macrophages.

This phenomenon is called antibody-dependent enhancement of infection. FIPV is also a virus that infects macrophages. In addition, onset of FIP has been reported to be enhanced by this above-mentioned antibody-dependent enhancement of infection. The main objective of conventional vaccine development strategies has been to induce neutralizing antibodies. Directly applying such vaccine development strategies to FIP can be thought to be difficult.

However, research into and development of FIP vaccines has mainly been carried out based on conventional vaccine development strategies. Thus, preferable results were not always obtained when the mechanism of antibody-dependent enhancement of infection for FIP was not fully considered. Previous attempts at vaccine development are described below.

To date, the protective effects of various vaccines against infection have been investigated, including FIPV-inactivated vaccine, live FIPV-attenuated vaccine, and such. However, in all cases, a sufficient effect could not be obtained. Rather, infection was enhanced.

In 1980, Pedersen and Boyle showed that intraperitoneal inoculation of highly virulent FIPV causes severe symptoms to appear more rapidly in kittens already positive for FIPV antibody, or in kittens passively immunized with the serum of antibody-positive cats or the purified IgG thereof, compared to antibody-negative kittens (Pedersen NC, Boyle JF. Immunologic phenomena in the effusive form of feline infectious peritonitis. Am J Vet Res. 1980 Jun; 41(6) :868-76).

In 1981, in the same way, Weiss and Scott passively immunized SPF kittens with the serum of infected cats, and then intraperitoneally inoculated them with the virus. In their results also, FIPV infection readily occurred in cats passively immunized with antibodies, with their body temperatures increasing 24 hours after inoculation and continuing thereafter until death. Their survival period was nine to ten days, clearly shorter than the 14 to 52 days for the control antibody-negative cats (Weiss RC, Scott FW. Antibody-mediated enhancement of disease in feline infectious peritonitis: comparisons with dengue hemorrhagic fever. Comp Immunol Microbiol Infect Dis. 1981; 4(2):175-89).

Thereafter, other researchers also confirmed antibody-dependent enhancement of infection. At present, antibody-mediated enhancement of FIPV infection is widely recognized among many researchers as a major obstacle to FIP vaccine development.

As described above, FIPV is composed of three major structural proteins: the N, M, and S proteins. The results of studies to date have revealed that neutralizing epitopes and infection-enhancing epitopes coexist on the S protein, and that both are closely related. In conventional vaccine development strategies, the development of vaccines that use the S protein as the antigen is attempted first. However, infection-defense vaccines that utilize the S protein simultaneously comprise epitopes that enhance infection, and are always accompanied by the risk of enhancing infection.

In fact, in previously produced recombinant vaccines, a gene encoding the antigenic determinant (S protein), which was associated with neutralization of the type II virus, was inserted into a vaccinia virus. However, this did not prevent infection, but rather enhanced FIP (Vennema H, de Groot RJ, Harbour DA, Dalderup M, Gruffydd-Jones T, Horzinek MC, Spaan WJ. Early death after feline infectious peritonitis virus challenge due to recombinant vaccinia virus immunization. J Virol. 1990 Mar; 64(3):1407-9).

Meanwhile, a live virus vaccine derived from a temperature-sensitive virus strain was developed by an American research group. The vaccine aims to increase local immunity by intranasal inoculation of a temperature-sensitive virus strain (Gerber JD, Ingersoll JD, Gast AM, Christianson KK, Selzer NL, Landon RM, Pfeiffer NE, Sharpee RL, Beckenhauer WH. Protection against feline infectious peritonitis by intranasal Inoculation of a temperature-sensitive FIPV vaccine. Vaccine. 1990 Dec; 8(6):536-42). Since the temperature-sensitive strain cannot grow at high temperatures, its proliferation sites are expected to be limited, even if it enters the body.

This vaccine has already been clinically applied in America and Europe. However, evaluation of its efficacy and safety differs depending on the researcher. It is reported that in some cases when vaccinated cats are experimentally challenged, depending on the amount of the challenging virus, the infection may instead be enhanced (Scott, FW., Corapi, WV., and Olsen, CW. Evaluation of the safety and efficacy of Primucell-FIP vaccine. Feline Hlth Top. 1992, 7: 6-8; Scott, FW., Corapi, WV., and Olsen, CW. Independent evaluation of a modified live FIPV vaccine under experimental conditions. Feline Practice 1995, 23: 74-76).

Furthermore, recombinant vaccines in which the M protein- or N protein-encoding gene is inserted into a vaccinia virus or poxvirus are reported to be effective for preventing FIP to a certain degree (Vennema H, de Groot RJ, Harbour DA, Horzinek MC, Spaan WJ. Primary structure of the membrane and nucleocapsid protein genes of feline infectious peritonitis virus and immunogenicity of recombinant vaccinia viruses in kittens. Virology. 1991 Mar;181(1):327-35; Wasmoen TL, Kadakia NP, Unfer RC, Fickbohm BL, Cook CP, Chu HJ, Acree WM., Protection of cats from infectious peritonitis by vaccination with a recombinant raccoon poxvirus expressing the nucleocapsid gene of feline infectious peritonitis virus. Adv Exp Med Biol. 1995;380:221-8). However, since these vaccines are recombinant live vaccines, many problems, including safety, must be cleared up for their field application.

Therefore at present, vaccines sufficiently satisfactory in terms of their protective effect against FIPV infection and safety have not yet been developed.

### Disclosure of the Invention

An objective of the present invention is to provide vaccines that are useful for prevention or treatment of FIP.

For example, inferring from conventional methods for developing vaccines against viral infection, two effective targets can be considered as mechanisms for preventing FIP. First, after oral or nasal infection, FIPV passes the mucosal barrier, and spreads throughout the body via macrophages. Passage of the mucosal barrier and expression of FIP symptoms depend on the infective dose and virulence level of the virus. Therefore, the first target for prevention of FIPV infection is the suppression of viral growth in the mucosa, and of viral entry into tissues.

Next, cellular immunity must be elevated to prevent the growth of FIPV that has passed through the mucosal barrier, and that persistently infects phagocytes. That is, the immune system ideally removes the virus-infected cells. This is the second target for prevention of FIPV infection. In fact, cellular immunity is reported to be elevated in survived cats against challenge with virulent FIPV.

Based on this kind of background, out of the viral component proteins, the present inventors focused on the N protein, which does not include infection-enhancing epitopes. Generally, however, since the N protein does not exist on the surface of viral particles and infected cells, immunization with the N protein alone will not enhance the infection. On the other hand, complete prevention of the infection was predicted to be difficult. In fact, there is a report of the use of a recombinant vaccine in which the gene encoding the N protein of type II FIPV is expressed in a vaccinia virus (US 5811104). However, data supporting an infection-preventing effect was not obtained in this report. Therefore, these results showed that even if a type II FIPV N protein is used as an antigen, development of a vaccine with an excellent effect in preventing infection or onset is difficult.

On the other hand, there are no reports of vaccines that utilize type I N protein. This may be due to reasons such as the following: First, since type I FIPV proliferates slowly during tissue cultivation, it can be said to be a difficult experimental material to handle. Furthermore, type I FIPV is less pathogenic for cats than type II FIPV, resulting in a low rate of FIP onset. For these reasons, the design of type I FIPV infection experiments is difficult, and thus the use of type I FIPV as a material for FIP vaccine research is accompanied by difficulties. However, these reasons do not negate the importance of developing vaccines effective against type I FIPV. In fact, clinically, the cause of 70% or more of FIP is type I virus infections, and isolation of type II virus is relatively low.

Therefore, the present inventors considered that in order to obtain vaccines expected to have practical effects, the use of type I virus-derived antigens would be an important condition. They also continued to search for type I virus-derived antigens that could be used as vaccine materials. As a result, the present inventors found that vaccines that use, as the antigen, an N protein comprising a specific amino acid sequence derived from type I viruses, may provide preventive effects against a wide range of FIPVs, thereby completing this invention. More specifically, the present invention relates to the following vaccines for prevention or treatment of FIP, and methods for preventing or treating FIP. Furthermore, the present invention relates to methods of testing for FIP, and to testing reagents for FIP.

The subject matter of the invention is defined in the claims. Preferred embodiments are described in the following.

Thus, the present invention relates to the use of polynucleotides of any one of the following a) to e),
a) a polynucleotide comprising a coding region of the nucleotide sequence of SEQ ID NO: 1;
b) a polynucleotide comprising a nucleotide sequence that encodes the amino acid sequence of SEQ ID NO: 2;
c) a polynucleotide comprising a nucleotide sequence with 93% or more homology to a nucleotide sequence of a coding region of the nucleotide sequence of SEQ ID NO: 1;
d) a polynucleotide comprising a nucleotide sequence with 93% or more homology to the nucleotide sequence encoding the amino acid sequence of SEQ ID NO: 2; and
e) a polynucleotide encoding a continuous amino acid sequence comprising 45 or more amino acid residues, selected from an amino acid sequence encoded by the polynucleotide of a) or b).
or proteins comprising the amino acid sequence encoded by these polynucleotides, in the production of vaccines for treating and/or preventing feline infectious peritonitis. Furthermore, the present invention relates to the use of antibodies that can bind to proteins comprising the amino acid sequence encoded by the polynucleotide of any one of a) to e), in the production of antibody formulations for treating and/or preventing feline infectious peritonitis,

The nucleotide sequence of SEQ ID NO: 1, and the amino acid sequence (SEQ ID NO: 2) encoded by this nucleotide sequence, are derived from the KU-2 strain of type I FIPV. The nucleotide sequence of the KU-2 gene, and the amino acid sequence encoded by the gene, are already known (Motokawa, K. et al. Microbiol. Immunol., 40/6, 425-433, 1996). However, it is not known that it is possible to prevent and treat FIP using this gene.

As mentioned above, the nucleotide sequences of strains classified as type I FIPV have low homology. Table 1 shows the homology among representative FIPV strains for which the nucleotide sequences of the genes encoding the N protein have been identified, and other closely related viruses. For example, the N protein of KU-2 is less than 92% homologous with the N proteins of other strains. This is about the same degree of homology as between the N proteins of type II and feline enteric coronaviruses.

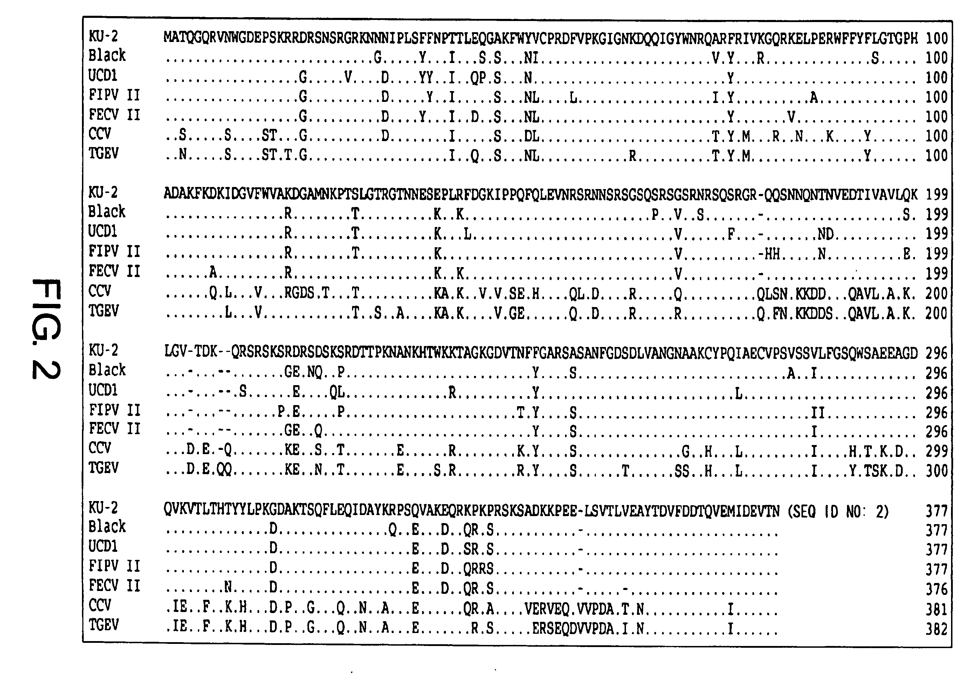

Accordingly, type I viruses vary greatly. Fig. 2 shows the results of comparing the amino acid sequences of N proteins. Fig. 2 shows the differences between the amino acid sequences of each of the viruses, and shows that the amino acid sequence of the KU-2 strain is different from the other strains. Therefore, the preventive and therapeutic effects of each vaccine prepared from any one of the type I viruses are predicted to differ, depending on the viral strain. Contrary to predictions based on such conventional findings, the present invention is based on a novel finding that the viral antigen derived from a specific strain is useful in producing vaccines that are very safe and effective against a wide variety of strains.

Specifically, the present invention relates a subunit vaccine for treating and/or preventing feline infectious peritonitis, wherein said vaccine comprises a protein comprising an amino acid sequence encoded by a polynucleotide of any one of a) to e) as the active ingredient:
a) a polynucleotide comprising a coding region of the nucleotide sequence of SEQ ID NO: 1;
b) a polynucleotide comprising a nucleotide sequence that encodes the amino acid sequence of SEQ ID NO: 2;
c) a polynucleotide comprising a nucleotide sequence with 93% or more homology to a nucleotide sequence of a coding region of the nucleotide sequence of SEQ ID NO: 1;
d) a polynucleotide comprising a nucleotide sequence with 93% or more homology to the nucleotide sequence encoding the amino acid sequence of SEQ ID NO: 2; and
e) a polynucleotide encoding a continuous amino acid sequence comprising 45 or more amino acid residues, selected from an amino acid sequence encoded by the polynucleotide of any one of a) to d).

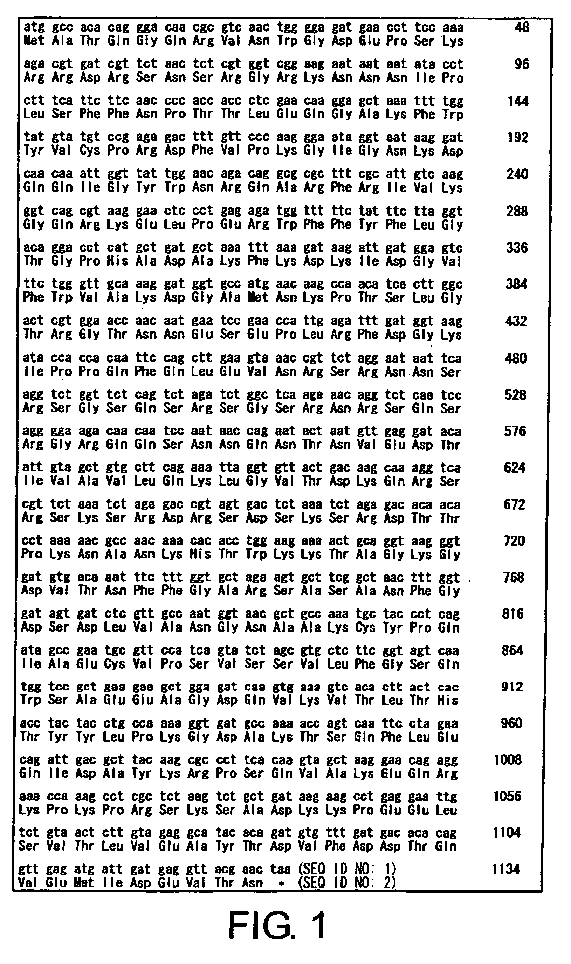

As an active ingredient, the vaccines of this invention may comprise the N protein derived from the FIPV strain KU-2. The amino acid sequence of the N protein of the KU-2 strain is shown in SEQ ID NO: 2, and the nucleotide sequence encoding this amino acid sequence is shown in SEQ ID NO: 1 and Fig. 1. In addition, a protein comprising an amino acid sequence encoded by:
c) a polynucleotide comprising a nucleotide sequence with 93% or more homology to a nucleotide sequence of a coding region of the nucleotide sequence of SEQ ID NO: 1; or
d) a polynucleotide comprising a nucleotide sequence with 93% or more homology to the nucleotide sequence encoding the amino acid sequence of SEQ ID NO: 2
may be used as an active ingredient in the vaccines of this invention. Known FIPV N proteins classified into type I are all less than 93% homologous with the N protein of KU-2 strain.

In general, the immunological characteristics of proteins comprising highly homologous amino acid sequences are similar. In the present invention, a preferable amino acid sequence is an amino acid sequence with normally 93% or more, more preferably 95% or more, and even more preferably 98%, 99% or more homology to the amino acid sequence of SEQ ID NO: 2.

Methods for determining amino acid sequence homologies are well known. For example, the BLAST algorithm by Karlin and Altschul is a representative algorithm for determining amino acid sequence homology (Proc. Natl. Acad. Sci. USA 90:5873-5877, 1993). The BLASTX program can realize this algorithm (Altschul et al. J. Mol. Biol. 215:403-410, 1990). When using BLASTX to analyze the amino acid sequence, the parameters are set at score= 50 and wordlength= 3, for example. Alternatively, when using the BLAST and Gapped BLAST programs, the default parameters for each program may be used.

For example, in Example 2, the homologies as shown in Table 1 were calculated using Maximum Matching, of the genetic information processing software, GENETYX (Takashi K. and Gotho O. (1984) J. Biochem. 92:1173-1177). The parameters in this method are as follows:
Matching condition: matches=-1, mismatches= 1, gaps= 1, *N+= 2

Alternatively, homologies can be determined based on the Lipman-Pearson method (Lipman DJ and Pearson WR (1985) Science 227:1435-1441) . The parameters used in this method are as follows: Unit size to compare= 2 (amino acid) or 5 (nucleotide)

With respect to the amino acid sequences of the proteins used as active ingredients in the vaccines of the present invention, ordinarily 25 or fewer, or 20 or fewer, and preferably 0 to 15, or more preferably 0 to 5, and even more preferably 0 to 3 mutant amino acid residues may be introduced into the amino acid sequence of SEQ ID NO: 2. Generally, to avoid losing the properties of subject proteins as much as possible, the amino acids used for the substitution preferably have properties similar to the amino acids to be substituted. This type of amino acid substitution is called conservative substitution.

For example, since Ala, Val, Leu, Ile, Pro, Met, Phe, and Trp are all classified into non-polar amino acids, they have similar properties. Uncharged amino acids include Gly, Ser, Thr, Cys, Tyr, Asn, and Gln. Examples of acidic amino acids are Asp and Glu. Basic amino acids include Lys, Arg, and His.

Furthermore, a vaccine of this invention may comprise, as an active ingredient, a protein comprising e), which is a continuous amino acid sequence comprising 45 or more amino acid residues selected from an amino acid sequence encoded by the polynucleotide of any one of a) to d). The active ingredient of the vaccine does not have to maintain the entire structure of the antigen protein, as long as it can immunologically stimulate the immunocompetent cells. In general, immunostimulation by polypeptides comprising 15 or more amino acid residues can be detected without difficulty. On the other hand, amino acid sequences comprising 20 or 30 continuous amino acids are considered to constitute sequences unique to this protein. In particular, amino acid sequences comprising more than 45 amino acid residues constitute amino acid sequences specific to SEQ ID NO: 2. Therefore, proteins comprising partial amino acid sequences that satisfy the condition of e) can provide an antigenic stimulation to immunocompetent cells that is the same as that of the amino acid sequences selected from the amino acid sequences encoded by the polynucleotides of a) to d). Preferable polypeptides of the present invention are polypeptides comprising a continuous amino acid sequence selected from the amino acid sequence of SEQ ID NO: 2, and comprising 45 or more, 50 or more, preferably 55 or more, or more preferably 65 or more amino acid residues.

The preferable partial amino acid sequences of the present invention may comprise, within the amino acid sequences encoded by the polynucleotides of a) to d), nucleotide sequences that are predicted to be epitopes. Those skilled in the art can predict epitopes based on test amino acid sequences. Epitopes are predicted according to various characteristics of the amino acid sequence. Information on hydrophilicity/hydrophobicity, electric charges, glycosylation sequences, disulfide bonds, protein secondary structures, T-cell antigenic sites, and such is used to predict the parameters. Protein secondary structure can be predicted by the Chou-Fasman method, Robson method, or such. Furthermore, T-cell antigenic sites are predicted based on IA patterns and Rothbard/Taylor patterns.

Alternatively, epitopes can be experimentally specified by their analysis using monoclonal antibodies. This method is based on the reactivity of antibodies against peptide fragments comprising partial amino acid sequences that constitute the amino acid sequence of the immunogen, to determine epitopes that recognize those antibodies. The amino acid sequences of the peptide fragments were designed to slightly overlap. This analysis method can determine which of the amino acid sequences of the protein used as the antigen was recognized by the immune system.

Proteins comprising amino acid sequences encoded by the polynucleotides of a) to d) induce an immune response that suppresses the growth of FIPV, and do not comprise epitopes that enhance infection. Therefore, vaccines that are very safe and have good preventive effects can be provided. Furthermore, protein fragments that comprise amino acid sequences constituting this protein epitope may induce a target immune response in a host in the same way, achieving FIP-preventive effects.

Preferred protein fragments in this invention are fragments which comprise activities that are immunologically equivalent to those of the proteins comprising the amino acid sequence of SEQ ID NO: 2. Immunologically equivalent means that when administered to a host, a protein fragment will induce the same immune response as when a protein comprising the amino acid sequence of SEQ ID NO: 2 is administered.

The protein fragments of this invention may be used alone, or in combination with fragments comprising different amino acid sequences. Therefore, combinations of fragments that show immunologically equivalent activities are included in this invention, even if each fragment itself is not immunologically equivalent. Furthermore, vaccines comprising fragments that can yield immunologically equivalent activity by combining auxiliary components other than protein fragments are included in this invention.

For example, by mixing specific adjuvants, protein fragments showing activities that are immunologically equivalent to that of a protein comprising an amino acid sequence of SEQ ID NO: 2 can be used in this invention. In addition, protein fragments that maintain immunologically equivalent activities by forming fusion proteins with appropriate carrier proteins can also be used in this invention.

The immune response of hosts against administered proteins can be compared based on, for example, the following indicators:
· antibody titer against the administered proteins;
· activation effect of cellular immunity; and
· level of biophylaxis function against the challenge of infectious source concerned.

Antibody titer against an administered protein can be measured using various methods of immunoassay. More specifically, antibody titers can be measured by applying test animal blood samples to microtiter plates solid phased with a protein comprising the amino acid sequence of SEQ ID NO: 2; and then reacting the microtiter plates with labeled antibodies against the immunoglobulin of the animals.

Furthermore, the effect of cellular immunity activation by administered proteins can be evaluated, for example, by *in vitro* measurement of the degree of T cell activation in the peripheral blood. More specifically, CTL assays are known as methods for measuring the aggressive activity of test T cells against target cells. Recently, methods for monitoring T-cell activation levels using cytokines or perforins as indicators are also being used. IL-2, γ-IFN, and such are used as indicators of T cell activation. Perforins are biomolecules involved in cytotoxicity. These indicators are measured using ELISA, real time PCR, ELISPOT method, and such. In general, such measurement techniques are simple compared to CTL assays, which require advanced cell culturing techniques.

Immunological equivalence can also be confirmed by actually administering individuals with a protein, challenging them with a pathogen, and then comparing their protection levels. Protection levels can be compared based on changes in body weight and temperature, days of survival, or such after inoculation with pathogens.

The polynucleotides of a) to e) of the present invention are not limited in origin. Naturally occurring polynucleotides, as well as artificially or spontaneously mutated polynucleotides are acceptable. Polynucleotides comprising artificially designed sequences are also acceptable.

Naturally occurring polynucleotides include, for example, polynucleotides of the KU-2 strain, and polynucleotides derived from mutant KU-2 strains. In addition, polynucleotides derived from FIPV, which comprises nucleotide sequences highly homologous to those of the KU-2 strain, may also be used. On the other hand, the artificially designed polynucleotides of the present invention may be polynucleotides in which the nucleotide sequence of the KU-2 strain is artificially mutated.

The polynucleotides of the present invention can be prepared, for example, by using conventional hybridization techniques (Sambrook J., Fritsch, E. F., and Maniatis T. Molecular cloning: A Laboratory Manual (2nd edition). Cold Spring Harbor Laboratory Press, Cold Spring Harbor). Polymerase chain reaction techniques can be used to isolate DNAs (Sambrook J. , Fritsch, E. F., and Maniatis T. Molecular cloning: A Laboratory Manual (2nd edition) . Cold Spring Harbor Laboratory Press, Cold Spring Harbor).

Those skilled in the art can screen and isolate virus-derived DNAs or such using hybridization methods and PCR methods. The nucleotide sequences of probes necessary for hybridization methods and primers necessary for PCR can be designed, for example, based on the cDNA sequence of the N protein of the KU-2 strain (SEQ ID NO: 1).

Methods for mutating amino acids are well known. For example, virus libraries comprising mutant viruses, DNA libraries encoding mutant N proteins, and such are prepared to screen and isolate DNAs that encode preferred amino acid sequences. Alternatively, mutant viruses can be screened from nature. Furthermore, site-directed mutagenesis can be carried out using known genetic engineering techniques. In site-directed mutagenesis, methods such as the SOE (splicing by overlap extension)-PCR method (Ho, S.N., Hunt, H.D., Horton, R.M., Pullen, J.K., and Pease, L.R. (1989) Gene 77, 51-59), and the Kunkel method may be used (Kunkel, T.A. (1985) Proc. Natl. Acad. Sci. USA; 82(2):488-92).

Proteins which can be used as active ingredients of the vaccines of this invention can be obtained by conventional methods. For example, the proteins can be expressed by inserting above-described polynucleotides of any one of a) to e) into appropriate expression vectors; and introducing the vectors into host cells. Examples of hosts are bacteria, yeast, insect cells, mammalian cells, and mammals. More specific examples are, *Escherichia coli* for bacteria, *Shizosaccharomyces pombe* for yeast, and CHO cells, COS cells, and such for mammalian cells.

Well-known vectors may be used as the vectors that can be introduced into these hosts. Of these, expression systems using insect cells and baculovirus vectors are useful for preparing the vaccines of this invention.

A baculovirus (Autographa californica nuclear polyhedrosis virus; AcNPV), uses insects as a host and comprises a double-stranded circular DNA genome. Polyhedra containing many viral particles are produced in the nuclei of infected cells, and serve as the infection source. The expression of the polyhedral protein, "polyhidrin", which is one of the proteins constituting a polyhedron, is regulated by a powerful promoter. The baculovirus expression system utilizes the activity of this powerful polyhidrin promoter.

The polyhidrin gene shows extremely high expression levels in the later stage of infection. However, in cultured cells, it is not an essential protein for viral proliferation. Therefore, if the polyhidrin gene, which is downstream of the polyhidrin promoter, is substituted with an exogenous gene, high expression of the exogenous gene can be expected, as for polyhidrin. More specifically, the baculovirus expression system is constructed according to the following steps:
· subcloning exogenous genes into transfer vectors;
· preparing recombinant viruses; and
· expressing proteins.

Direct insertion of an exogenous gene into an approximately 130 kb baculovirus genome is difficult. Therefore, the exogenous gene is inserted into a transfer vector consisting of an approximately 10 kb plasmid. The transfer vector and viral DNA are simultaneously transfected into host cells, and then the exogenous gene is inserted into the virus using homologous recombination.

Commercially available vectors for baculovirus recombination can be used as the transfer vectors. For example, pVL1392 and pVL1393 (both from Pharmingen), as well as pPAK8 and pPAK9 (both from Clontech) are commonly used transfer vectors. In addition, vectors comprising additional various functions are commercially available, such as vectors to which a signal sequence can be added to the N terminus, vectors to which a histidine tag can be added, or vectors in which a number of promoters are inserted. Any of these commercially available vectors can be used for this invention.

In the culture supernatant of infected cells, both successfully recombined viruses and viruses that have not undergone recombination are produced. Thus, recombinant viruses can be selected as necessary. Recombinant viruses can be selected by utilizing plaque formation. More specifically, recombinant viruses can be isolated based on the fact that cells infected with recombinant viruses cannot form polyhedra, and thus form clear plaques. In contrast, viruses that have not undergone recombination express large amounts of polyhedra, and thus form white plaques. The titer of obtained recombinant viruses can be amplified as necessary by repeated infection.

Due to the low recombination efficiency of the DNA of wild-type cyclic AcNPV, instead, mutant viruses with improved recombination efficiency have been put to practical use. For example, commercially available mutant baculoviruses can be expected to have high recombination efficiencies, close to 100%. As a result, the step of plaque purification is unnecessary. More specifically, mutant baculoviruses such as Baculo Gold^{™} Linearized Baculovirus (Pharmingen) and Bsu 36 1-digested BacPAK6 viral DNA (Clontech) are commercially available.

Insect cells are used for recombination of baculoviruses and protein expression. The most common host cells are insect cell strain, Sf9. Sf9 can be purchased from Pharmingen, Clontech, ATCC, or such. In addition, insect cells such as Sf21 (Invitrogen, Pharmingen) or High Five^{™} (Invitrogen) may be used in this invention.

These insect cells can be maintained in appropriate media. Commercially available media such as Grace Insect Medium, TMN-FH Insect Medium, or Excell 400 may be used as the media. Generally, insect cells are cultured at around 27°C, and unlike animal cells, do not require CO₂. Sf9 grows well in both monolayer cultures and suspension cultures.

Methods for transfecting transfer vectors and baculovirus DNAs into insect cells are well known. Specifically, transfer vectors and baculovirus DNAs are infected into cells using Lipofectin Reagent. If cultivation is continued after infection, recombinant viruses are produced in the culture supernatant. The viruses in the supernatant are amplified as necessary, and then infected into a large number of insect cells to express large amounts of the exogenous genes. Cells expressing the genes can be harvested, and the expression products of the exogenous genes can be purified.

Baculovirus expression systems are generally considered to have the following advantages over expression systems that use *E. coli* as the host:
· expression amounts are large;
· expression levels are also relatively high for high molecular-weight proteins;
· post-translational modification occurs, as for expression in animal cells; and
· the expression and reconstitution of a number of types of proteins is possible.

On the other hand, for example, vectors carrying the replication gene necessary for replication in *E. coli* (ColE1 ori), the replication gene that supports expression in mammalian cells (SV40 ori), and the SV40 early promoter may be used as animal cell expression vectors. The above-described polynucleotides a) to e) are inserted downstream of the early promoter of such expression vectors, and the resulting vectors are cloned in to *E. coli*. The cloned expression vectors are collected and transfected into appropriate animal cells (such as simian kidney-derived COS cells) by calcium phosphate precipitation methods or liposome methods, and then N proteins can be expressed in the animal cells.

Proteins expressed as described above can be purified by conventional methods to obtain pure proteins. For purification, methods can be applied whereby N proteins are expressed as fusion proteins with each type of binding protein; and then purified using affinity chromatography. Well-known examples of such purification methods include systems for purifying histidine-tagged fusion proteins by adsorption through a nickel column. N proteins collected by affinity chromatography and such can be further purified using ion exchange chromatography.

Proteins used in the vaccines of this invention can be obtained by production methods using genetic recombination, as well as from cultured cells infected with FIPV. Primary cells or established cell lines derived from mammals may be used to culture FIPV. In particular, cat-derived cells are preferable for culturing FIPV. Established feline cell lines are, for example, fcwf4 cells and CRFK cells. These cells can be obtained from cell banks. Methods for culturing FIPV using established feline cell lines are well known (Hohdatsu T, Sasamoto T, Okada S, Koyama H. Antigenic analysis of feline coronaviruses with monoclonal antibodies (MAbs): preparation of MAbs which discriminate between FIPV strain 79-1146 and FECV strain 79-1683.Vet Microbiol. 1991 Jun;28(1):13-24). Alternatively, cells derived from other animals, such as pigs or dogs, may also be used.

Furthermore, the proteins necessary for vaccines can be chemically synthesized. Synthesis methods in which amino acids are sequentially linked to form oligopeptides that comprise a subject amino acid sequence are well known. In particular, protein fragments consisting of a relatively short amino acid sequence can easily be chemically synthesized. Proteins consisting of long amino acid sequences, which are difficult to chemically synthesize, can be synthesized by linking oligopeptides to each other.

The obtained N proteins can be used as vaccines as they are, and may alternatively be formulated according to pharmaceutical formulations. For example, formulation can be carried out by appropriately combining the proteins with pharmaceutically acceptable carriers or media, more specifically, with sterilized water and physiological saline, vegetable oil, emulsifiers, suspension, surfactants, stabilizers, and such. Adjuvants can be appropriately mixed with the vaccines of this invention.

The function of a vaccine of this invention can be enhanced by its combination with any adjuvant. Examples of adjuvants that may be used in the present invention include the following types of components:
inorganic substances such as aluminum salts;
microorganisms or microorganism-derived substances such as
   · BCG
   · muramyl dipeptide
   · Bordetella pertussis
   · pertussis toxin
   · chloera toxin;
surfactants such as
   · saponin
   · deoxycholate; and
squalene and related substances.

Adjuvants may be used alone or in combination with a number of substances. Vaccine specialists can determine appropriate adjuvant combinations by experimentation. Depending on the type of adjuvant, those that mainly stimulate humoral immunity, and those that mainly stimulate cellular immunity are known. For example, aluminum phosphate is known as an adjuvant that mainly stimulates humoral immunity. On the other hand, saponins such as Quil A and QS-21, pertussis toxin, cholera toxin, and such are known to easily stimulate cellular immunity. Adjuvants appropriate for use in combination with particular antigens can be selected while referring to such information.

Furthermore, the present invention relates to the above-described vaccines for treating and/or preventing feline infectious peritonitis, which comprise a polynucleotide of any one of a) to e) as an active ingredient. As mentioned above, proteins comprising an amino acid sequence encoded by a polynucleotide of any one of a) to e) are useful as vaccines for treating and/or preventing feline infectious peritonitis. Therefore, the effect of administering a vaccine of this invention can be expected to be the same as when an above-described polynucleotide of any one of a) to e) is introduced into a living body and expressed *in vivo.*

In the vaccines of the present invention, the aforementioned polynucleotides are introduced into a living body in an expressible state. To accomplish this, for example, vectors in which the above-mentioned polynucleotides are inserted downstream of the expression regulatory region functioning in the host can be introduced into a living body. By using a drug-sensitive promoter as the expression regulatory region, expression of the subject gene can be induced by administering the drug. For example, kanamycin-sensitive promoters are known to be such promoters. Alternatively, by using a promoter that induces site-specific expression, the expression site of a subject gene can be specified.

The technique of administering antigen-encoding genes to a living body, expressing the genes *in vivo,* and then using the antigens as vaccines is called "genetic vaccination". In genetic vaccination, genes encoding the antigens to be expressed are introduced into living bodies with or without appropriate carriers. Protein expression is known to be induced by the mere intramuscular injection of a sufficient amount of a DNA suspended in an appropriate buffer. When using a carrier, vectors and artificial carriers are used.

Viral vectors such as vaccinia virus can be used as the vectors. Vaccinia virus is a *Poxviridae* virus, and comprises a 186 kb DNA genome. As a vaccine against small pox, Vaccinia virus has already been inoculated into many people, and the word "vaccination" is derived from its name. Since vaccinia virus is transcribed and replicated in the cytoplasm, without translocation to the nucleus, there is little risk that the introduced gene will be integrated into the host genome. Therefore, also from a scientific viewpoint, vaccinia virus can be a very safe vector. In addition, the vaccinia virus vector is said to have a stronger stimulatory activity towards cellular immunity than towards humoral immunity. The characteristic of easily inducing cellular immunity is advantageous for the prevention or treatment of FIP, in which cellular immunity is very important.

In addition to vaccinia virus, adenoviruses, adeno-associated viruses, retroviruses, and such have been used as vectors for gene therapy. Any of these viral vectors can be used in the present invention.

Besides viral vectors, genetic vaccination using artificial carriers has been also tested. Examples of artificial carriers are liposomes and gold colloids.

For example, positively charged liposomes are adsorbed to negatively charged DNAs. When administered to a living body, the liposomes to which DNAs are adsorbed bind to the phospholipid layer of the cell surface, which carries a negative charge. Next, the DNAs are incorporated into the cell membrane by adsorption to the cell membrane, or by endocytosis. If vectors in which genes are inserted downstream of the promoters are used as the DNAs, these genes will be transcribed and translated into proteins in the cells to which the genes are transferred. Gold colloids are used in gene transfers that use a gene gun. More specifically, gold colloid particles coated with plasmids (naked DNA) are inoculated at high pressure using compressed gas. When the gold colloid particles enter the tissues, the genes are transferred into the cells. Gene transfer methods that use a gene gun enable high protein expression levels from the transfer of a small amount of DNA. Accordingly, a small amount of DNA can accomplish a sufficient vaccination effect.

The vaccines of this invention can be administered to animals by, for example, intraarterial injection, intravenous injection, or subcutaneous injection, as well as by intranasal, transbronchial, intramuscular, or oral methods well known to those skilled in the art. The dose varies depending on the body weight and age of the animals, the administration method, purpose of use, and such, and can be appropriately selected as necessary by one skilled in the art. For example, vaccines for cats are generally inoculated twice, eight weeks after birth or later, with a two to three-week interval.

The vaccines of the present invention are useful for preventing and/or treating FIP in cats and other Felidae animals. Based on epidemiological or virological data, the FIPV of wild Felidae animals are considered to be closely related to that of cats. Therefore, the vaccines of the present invention are also effective for Felidae animals.

In addition, it is provided the above-described antibody formulations for treating and/or preventing feline infectious peritonitis, which comprise antibodies that can bind to a protein comprising an amino acid sequence encoded by the polynucleotide of any one of a) to e) as the active ingredient.

As already described, the proteins comprising the amino acid sequences encoded by the polynucleotides of a) to d) induce immune responses that suppress growth of FIPV, but on the other hand, do not include epitopes that enhance infection. As a result, vaccines with excellent safety and preventive effects can be provided by using these proteins. Furthermore, the above-described antibodies capable of binding to proteins comprising the amino acid sequences encoded by the polynucleotides of a) to d) can accomplish therapeutic and/or preventive effects against feline infectious peritonitis by administration to hosts.

Administration of the antibodies that can bind to these proteins can place hosts in a condition similar to an immune response condition induced by protein administration for a short term. Accordingly, the administered antibodies suppress the growth of FIPV. Furthermore, since these proteins do not comprise epitopes that enhance infection, antibody-caused induction of infection can be avoided.

The antibody formulations for treating and/or preventing feline infectious peritonitis described herein can be obtained by immunizing animals using, as antigens, the above-described proteins comprising amino acid sequences encoded by the polynucleotides of a) to e). Any method may be used to obtain the antibodies. For example, antigens can be administered along with appropriate adjuvants to obtain antiserum from the blood of immunized animals. Alternatively, antibody producing cells of immunized animals are collected and cloned to obtain monoclonal antibodies. The antibody formulations described are preferably derived from the same species as the animals administered with the antibodies. Antibodies derived from the same species are safe, and can achieve therapeutic or preventive effects more easily.

Alternatively, antibody molecules derived from different species can be felinized using antibody engineering techniques. For example, a technique for constructing chimeric antibodies is known in which the constant region of an immunoglobulin is substituted with that of a feline immunoglobulin. More specifically, by linking a gene that encodes a variable region of an immunoglobulin of an arbitrary animal to a feline immunoglobulin gene, it is possible to make only the constant region of the immunoglobulin that of a cat-derived protein.

Immunoglobulin constant regions are more easily recognized as foreign than variable regions. Therefore, felinized constant regions can improve the safety of the immunoglobulins, and increase their stability *in vivo.* By using this technology to chimerize antibodies that have preferred binding affinities, immunoglobulins appropriate for administration to cats can be obtained.

Using this technology, chimeric antibodies in which mouse monoclonal antibodies against feline herpes virus and feline calicivirus have been felinized, are being commercialized (Umehashi M, Nishiyama K, Akiyama S, Kimachi K, Imamura T, Tomita Y, Sakaguchi S, Makino H, Shigaki T, Shinya N, Matsuda J, Tokiyoshi S. Development of Mouse-cat chimeric antibodies against feline viral rhinotracheitis and feline calicivirus infection. Sci. Rep. Chemo-Sero-Therap. Res. Inst., 6:39-48 (1997)).

Furthermore, felinized antibodies can be also obtained by substituting the feline immunoglobulin hypervariable region with the hypervariable region of an antibody with a preferred binding affinity. The immunoglobulin variable region is composed of a complementarity determining region (CDR), which determines binding affinity with an antigenic determinant; and a frame region, which maintains the CDR. The structure of the CDR is highly variable, and it is also called the hypervariable region. On the other hand, the frame region is highly conserved. Since antigen binding affinity is determined mainly by the CDR, CDR substitution can also modify the binding affinity of an immunoglobulin.

More specifically, primers that anneal to the frame region are designed, and cDNAs encoding the CDR are obtained by PCR. By substituting the CDR of a feline immunoglobulin with the obtained cDNAs, a feline immunoglobulin that comprises a CDR derived from any type of immunoglobulin can be obtained. That is, a preferred binding affinity can be introduced to a feline immunoglobulin by substituting the CDR of the feline immunoglobulin with the CDR of any type of immunoglobulin with the preferred binding affinity.

The antibody formulations described may comprise, as an active ingredient, an intact immunoglobulin capable of binding to proteins that comprise an amino acid sequence encoded by the polynucleotides of a) to d), or variable regions thereof. The antigen binding activity of an immunoglobulin is maintained by the variable region. Therefore, the variable region alone can be used as an active ingredient. However, the constant regions have important immune response functions, such as complement binding activity, and binding activity to Fc receptors on lymphocytes. Therefore, antibody formulations in which immunoglobulin molecules equipped with constant regions are active ingredients are preferable as the antibody formulations of this invention.

In the antibody formulations described, the term "antibodies" includes not only immunoglobulins themselves, but also crude immunoglobulins. Therefore, immunoglobulin-containing fractions such as antisera are also included as the antibodies. Meanwhile, the term "immunoglobulin" is used to describe antibodies based on structural characteristics. Antibodies and immunoglobulins can be used as the antibody formulations described, as long as they contain immunoglobulins with the required binding affinity. Thus, they do not have to be purified or monoclonal antibodies.

Antibodies may be formulated without further treatment, or according to pharmaceutical formulations. For example, they can be formulated by appropriate combination with pharmaceutically acceptable carriers or vehicles; more specifically, with sterilized water and physiological saline, vegetable oils, emulsifiers, suspensions, surfactants, stabilizers or such.

The antibody formulations described can be administered to animals by, for example, intraarterial injection, intravenous injection, or subcutaneous injection, as well as by intranasal, transbronchial, intramuscular, or oral methods well known to those skilled in the art. The dose varies depending on the body weight and age of the animals, the administration method, purpose of use, and so on, and can be appropriately selected as necessary by one skilled in the art.

In addition, the present invention relates to methods for treating and/or preventing feline infectious peritonitis, which comprise the process of administering the vaccines of this invention, to cats at least once. The methods of this invention can be applied to all animals belonging to the Felidae family. Methods for preparing vaccines and antibody formulations, and methods for administration to animals are as described above.

The vaccines of this invention are useful for preventing and/or treating FIP in cats. In the present invention, the term "preventing FIP" refers to the action of inhibiting FIP onset by prophylactic administration to animals that have not been infected with FIPV, or to infected animals that have not developed the disease. The term "inhibiting onset" includes, for example, the following effects:
preventing onset itself;
delaying onset;
alleviating symptoms after onset;
promoting healing after onset;
improving survival rate after onset; and
suppressing the infectivity of a diseased individual to other individuals.

Furthermore, the term "treatment" in the present invention refers to the effect of alleviating disease symptoms by administration to an individual who has developed the disease. The term "alleviating symptoms" includes, for example, the following effects:
easing symptoms;
promoting healing; and
improving survival rate after onset.

In addition, the present invention relates to methods of testing for feline infectious peritonitis virus infections, which comprise the steps of incubating cat serum with a protein comprising an amino acid sequence encoded by a polynucleotide of any one of a) to e) ; and then detecting an antibody that binds to the protein. As indicated in Example 10,N proteins derived from KU-2 react strongly with the antisera of a wide range of FIPV strains. This supports the fact that the N proteins of KU-2 are highly useful as FIPV vaccines, and that they are also useful as diagnostic antigens.

Diagnosis of viral infection is generally carried out using the viral antigens and antiviral antibodies in the biological sample as indicators. In particular, since antiviral antibodies can be detected even after viral antigens have disappeared, they are important diagnostic indicators. Viral antigens are necessary for the detection of antiviral antibodies.

As described above, viruses belonging to type I FIPV show low structural similarity. Therefore, in order to detect the presence of anti-FIPV antibodies in test animals with certainty, different proteins may have to be prepared for each strain. Antibodies against structurally different antigens may not be detected by using a single protein. On the other hand, by using test methods based on this invention, antisera against different strains can be tested with sufficient sensitivity by using a single protein as the antigen. The test methods of this invention are therefore useful in screening for FIPV.

More specifically, it is provided methods of screening for feline infectious peritonitis virus infection, which comprise the steps of incubating cat serum with a protein comprising an amino acid sequence encoded by a polynucleotide of any one of a) to e) ; and then detecting an antibody that binds to the protein. The methods of screening for feline infectious peritonitis virus infection refer to methods of testing an unspecified number of cats to find cats that may have been infected with FIPV.

Methods for detecting antibodies using specific antigens are well known. For example, antigens are immobilized onto a solid phase, and antibodies that bind to these antigens can be detected by antibodies (secondary antibodies) that recognize these antibodies. Alternatively, antigen-specific antibodies can be detected by capturing antibodies included in the sample onto a solid phase; and then reacting with antigens. In either of these methods, the antibodies and antigens can be labeled to facilitate detection. Enzymes, fluorescent substances, luminescent substances, colored particles, radioactive isotopes, and such are used to label antigens and antibodies. Methods for binding proteins to labeling compositions are well known.

Additional known methods for detecting antibodies are those that use antibody-induced agglutinations of particulate carriers sensitized with antigens as indicators. Methods that use particle agglutination as an indicator are useful as methods for continuously testing large quantities of samples, since they do not require separation of the solid and liquid phases.

Individuals in which antibodies that bind to a protein comprising an amino acid sequence encoded by the polynucleotide of any one of a) to e) were detected are diagnosed as having been infected by FIPV. Alternatively, symptom severity can be diagnosed by tracing changes in antibody titer. For example, an increase of antibody titer occurs after viral replication. Therefore, when antibody titer increases in an individual, viral replication is very likely to be occurring along with it. If antibody titer decreases after symptoms are relieved, the individual is diagnosed as very likely to be recovering.

The present invention also relates to feline infectious peritonitis viral infection test reagents, which comprise a protein comprising an amino acid sequence encoded by the polynucleotide of any one of a) to e). The proteins comprising an amino acid sequence encoded by the polynucleotide of any one of a) to e) in the reagent of this invention can be immobilized onto solid phases or particles according to the various immunoassay formats. Furthermore, diagnostic kits can be produced by combining the proteins with labeled antibodies, additional reagents necessary for detecting the labels, a positive or negative control, and such, according to the various immunoassay formats.

### Brief Description of the Drawings

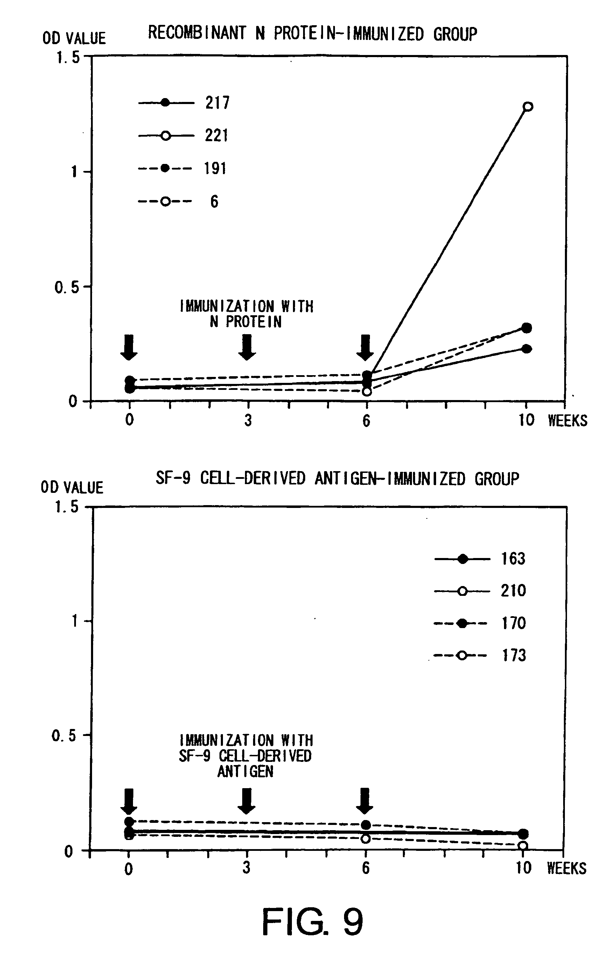

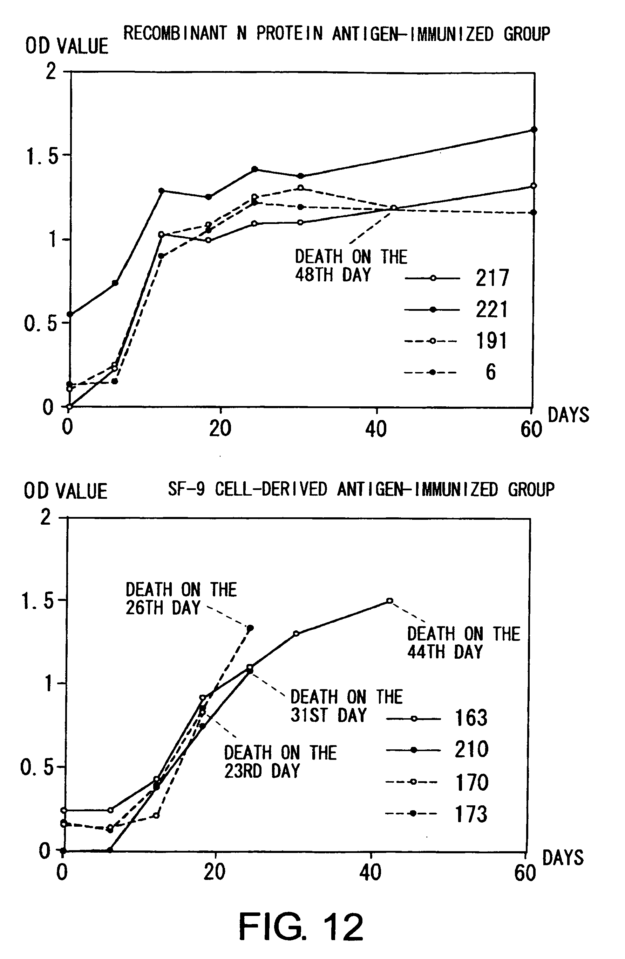

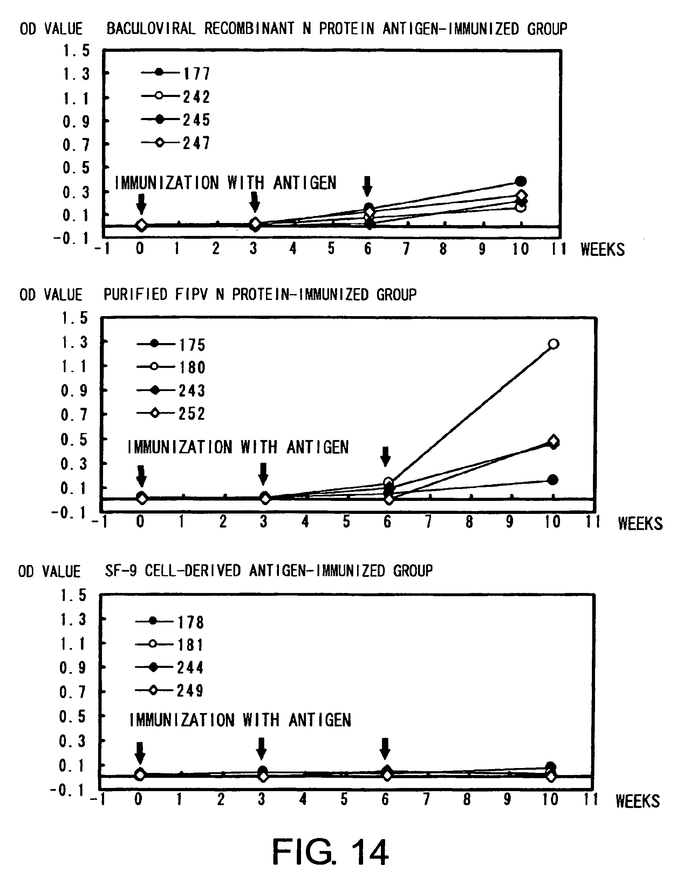

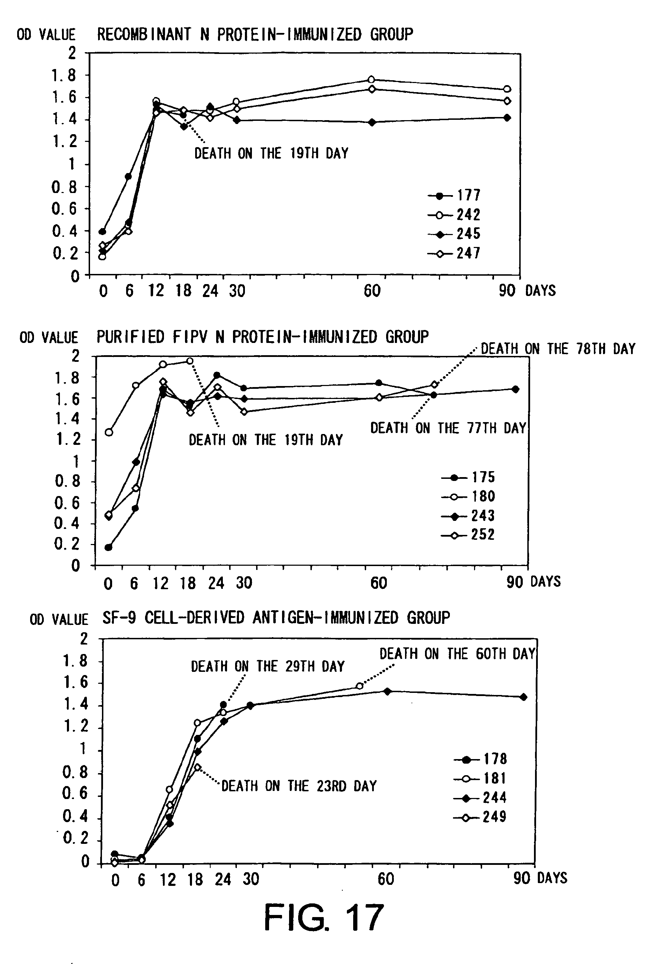

Fig. 1 shows the nucleotide sequence (SEQ ID NO: 1) and the amino acid sequence (SEQ ID NO: 2) of the N protein gene derived from type I FIPV strain KU-2.
Fig. 2 shows the results of aligning the N-protein amino acid sequences of FIPV with closely related viruses. In order from the top, each sequence shows the amino acid sequence of the N protein of type I FIPV strain KU-2 (SEQ ID NO: 2), Black, UCD1, type II FIPV strain 79-1146, type II FECV strain 79-1683, CCV Insvc1, and TGEV Purde. In this figure, "..." indicates that the amino acid residue is the same as that of KU-2, and "-" indicates a gap.
Fig. 3 shows a phylogenetic tree calculated from the amino acid sequences of the N genes of type I FIPV strain KU-2, Black, UCD1, type II FIPV strain 79-1146, type II FECV strain 79-1683, CCV Insvc1, and TGEV Purde. The numbers in the figure indicate evolutionary distance.
Fig. 4 is a photograph showing the results of using Western blotting to analyze the specificity of the baculoviral recombinant N protein antigen. The arrow in the figure indicates the band position for the N protein. "a", , "b", "c", "d", , "e", "f", and "g" indicate the baculoviral recombinant N protein antigen diluted eight, 16, 32, 64, 128, 256, and 512 times, respectively. "h" indicates the solubilized whole antigen of purified FIPV.
Fig. 5 is a photograph showing the results of using Western blotting to analyze the specificity of the purified FIPV-antigen-derived N protein. The arrow in the figure indicates the band position for the N protein. "a" indicates the purified FIPV-antigen-derived N protein, and "c" shows the solubilized whole antigen of purified FIPV.
Fig. 6 shows the result of using ELISA to analyze the specificity of the baculoviral recombinant N protein antigen. The horizontal axis shows the antigen dilution ratio, and the vertical axis shows optical density at 450 nm.
Fig. 7 shows the result of analyzing the specificity of the purified FIPV-antigen-derived N protein by ELISA. The x-axis shows the antigen dilution ratio, and the y-axis shows the optical density at 450 nm.
Fig. 8 shows the schedule of immunization test (1) and challenge test (1). Fig. 8 (a) shows the group immunized with baculoviral recombinant N protein, and Fig. 8(b) shows the group immunized with SF-9 cell-derived antigen.
   The upper panel of Fig. 9 shows the results of using ELISA to measure antibody titer after immunization of the baculoviral recombinant N protein. The lower panel shows the antibody titer after immunization with the SF-9 cell-derived antigen. The numbers in the panels correspond to the numbers of the cats used in the experiment. The x-axis shows the passage of time after immunization, and the y-axis shows optical density at 450 nm.

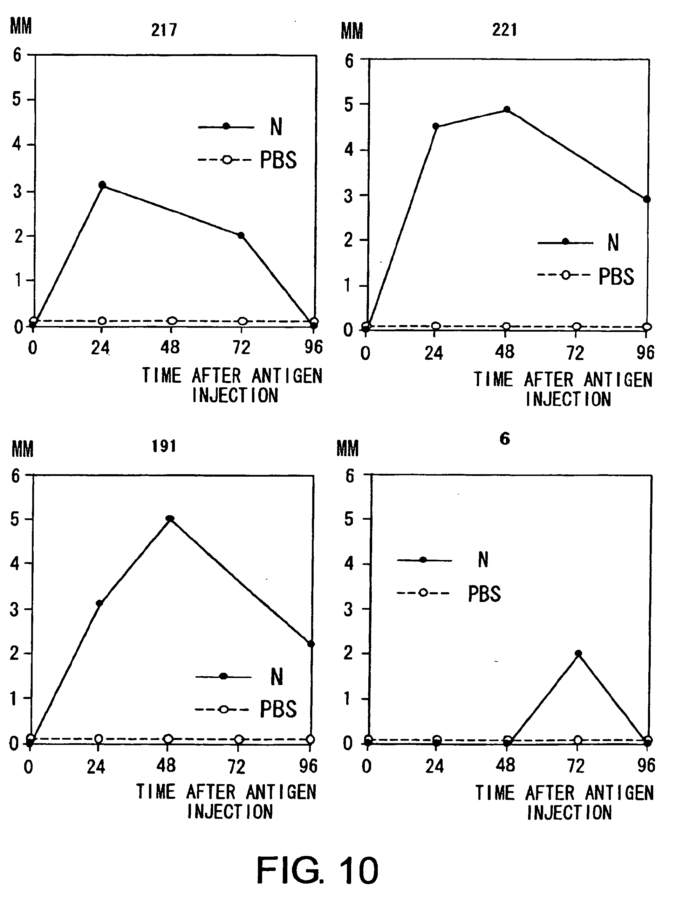

Fig. 10 shows delayed hypersensitivity in cats immunized with the baculoviral recombinant N protein. The numbers in the figure correspond to the numbers of the cats used in the experiment. The x-axis shows the time after antigen injection, and the y-axis shows the diameter of the range of swelling.

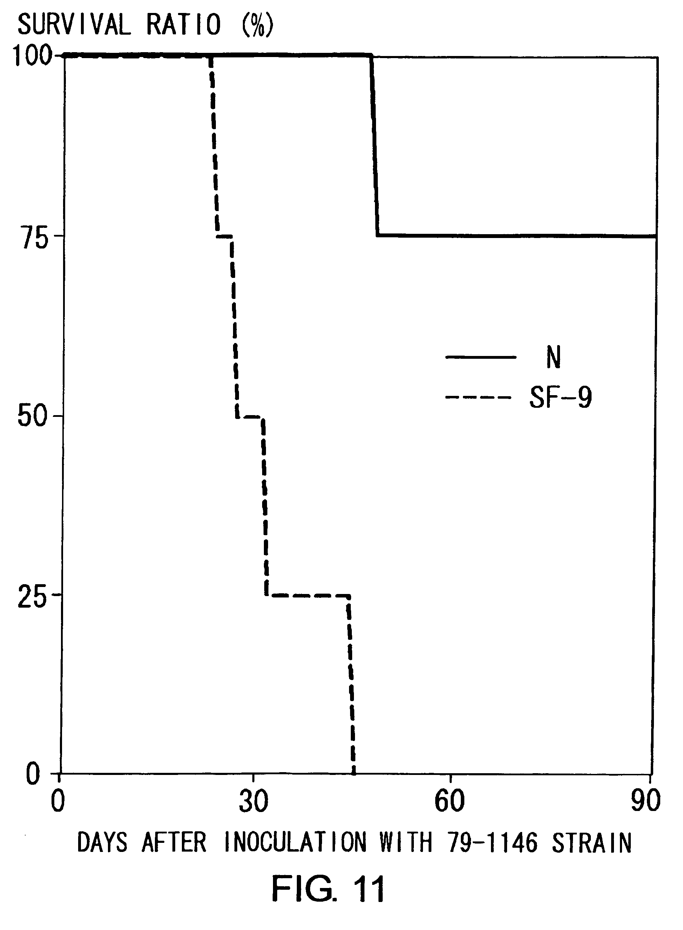

Fig. 11 shows changes in survival ratio after challenge with the FIPV79-1146 strain. The x-axis shows the number of days after inoculation of the 79-1146 strain, and the y-axis shows survival rate. The solid line shows the survival ratio of cats immunized with the baculoviral recombinant N protein, and the dashed line shows the survival ratio of cats immunized with the SF-9 cell-derived antigen.
   The upper panel of Fig. 12 shows the changes in anti-FIPV antibody titer after inoculating the 79-1146 strain to the baculoviral recombinant N protein-immunized group. The lower panel shows the change in anti-FIPV antibody titer after inoculating the 79-1146 strain to the SF-9 cell-derived antigen-immunized group. The numbers in the panels correspond to the numbers of the cats used in the experiment. The x-axis indicates the days after inoculation of the 79-1146 strain, and the y-axis indicates optical density at 450 nm.

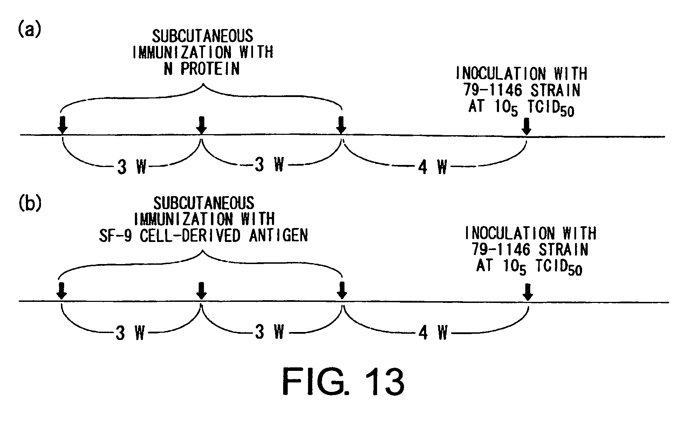

Fig. 13 shows the schedule of immunization test (2) and challenge test (2). Fig. 13(a) is the group immunized with the baculoviral recombinant N protein, and the group immunized with the purified FIPV N protein. Fig. 13 (b) indicates the group immunized with SF-9 cell-derived antigen.
   The upper panel of Fig. 14 shows the results of using ELISA to measure the antibody titer after immunization of the baculoviral recombinant N protein. The middle panel shows antibody titer after immunization of the purified FIPV N protein. The lower panel shows antibody titer after immunization of the SF-9 cell-derived antigen. The numbers in the figures correspond to the numbers of the cats used in the experiment. The x-axis indicates the passage of time after immunization, and the y-axis indicates optical density at 450 nm.

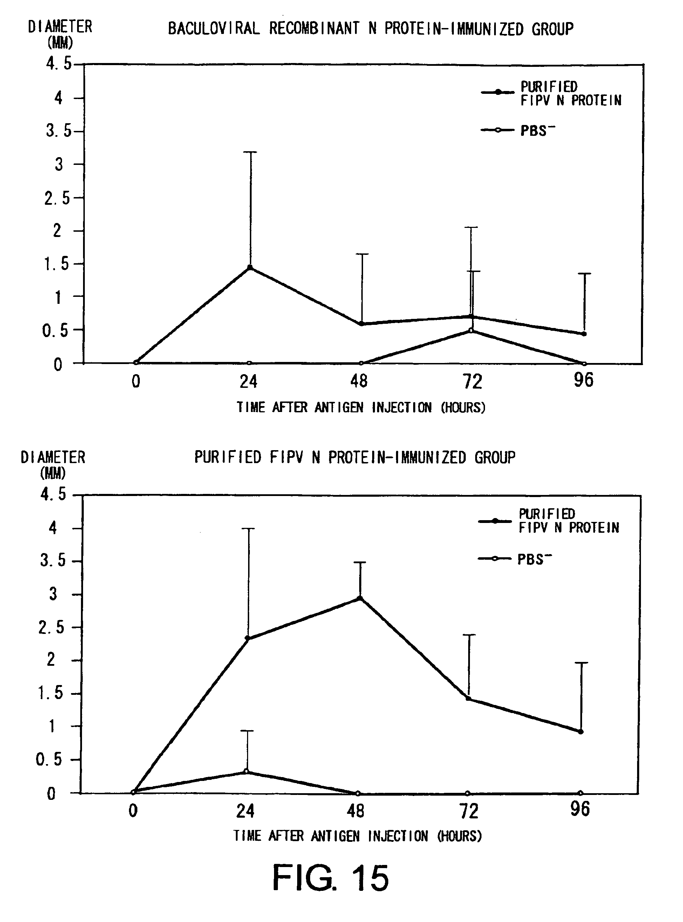

Fig. 15 shows delayed hypersensitivity in cats immunized with the baculoviral recombinant N protein and cats immunized with the purified FIPV N protein. The x-axis indicates the time after antigen injection, and the y-axis indicates the swelling diameter.

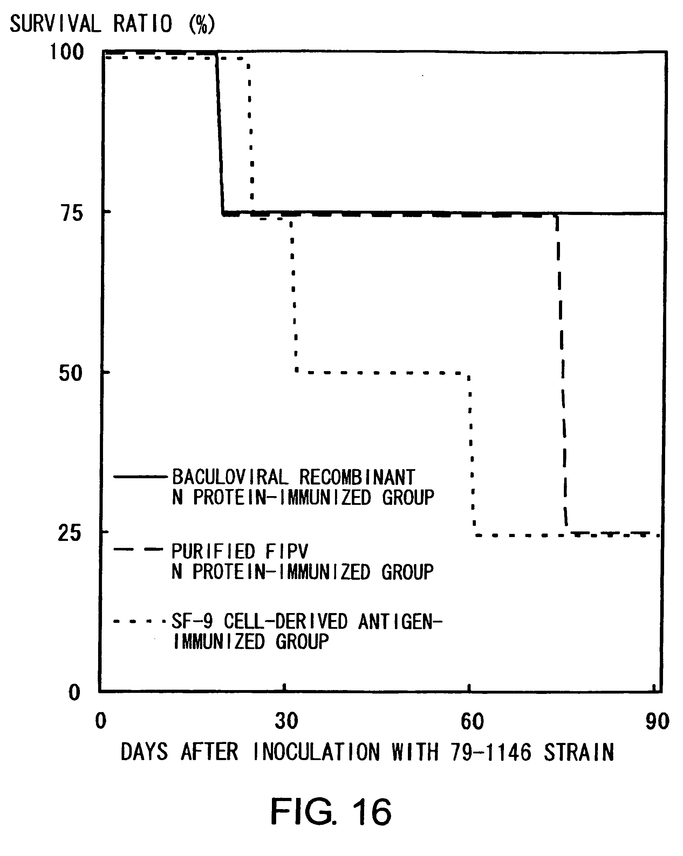

Fig. 16 shows changes in survival ratio after challenge with the FIPV 79-1146 strain. The x-axis shows the days after inoculation with the 79-1146 strain, and the y-axis indicates the survival ratio.
   The upper panel of Fig. 17 shows changes in anti-FIPV antibody titer after inoculating the 79-1146 strain to the baculoviral recombinant N protein-immunized group. The middle panel shows changes in anti-FIPV antibody titer after inoculating the 79-1146 strain to the purified FIPV N protein-immunized group. The lower panel shows changes in anti-FIPV antibody titer after inoculating the 79-1146 strain to the SF-9 cell-derived antigen-immunized group. The numbers in the figures correspond to the numbers of the cats used in the experiment. The x-axis indicates the days after inoculation of 79-1146 strain, and the y-axis indicates optical density at 450 nm.

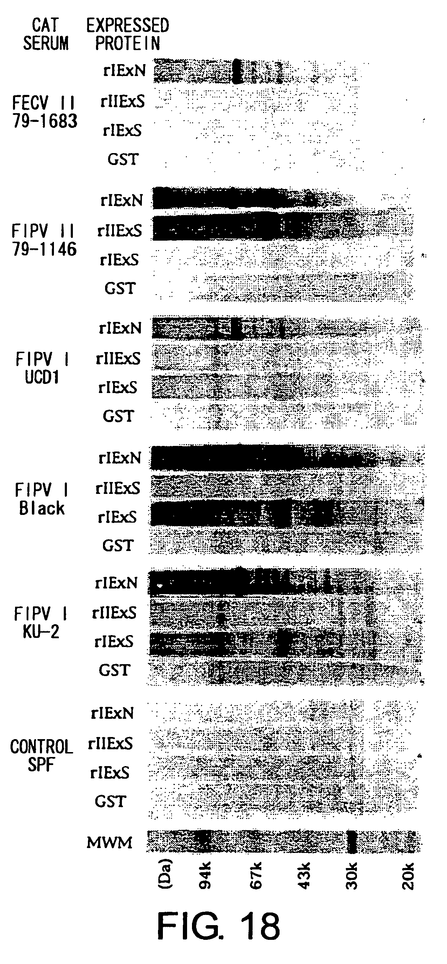

Fig. 18 is a photograph showing the results of a Western blotting assay to investigate the reactivity of the sera of feline coronavirus-infected cats against E. coli-expressed antigens. Each lane shows the result of reacting the serum of cats infected with the coronavirus strain indicated at the top of the lanes, using a filter blotted with the antigen indicated immediately above the lane.

### Best Mode for Carrying out the Invention

Herein below, the present invention is specifically described using Examples.

### [1] Purification of viral protein

### Origin of type I FIPV strain KU-2:

Vaccine antigens and recombinants were produced using the FIPV KU-2 strain, isolated at the Department of Veterinary Infectious Diseases, School of Veterinary Medicine and Animal Sciences, Kitasato University from cats that have developed FIP. This virus is classified as a type I FIPV.

### Origin of type II FIPV strain KU-1:

Purified N proteins were produced using the FIPV KU-1 strain, isolated at the Department of Veterinary Infectious Diseases, School of Veterinary Medicine and Animal Science, Kitasato University, from cats that have developed FIP. This virus is classified as a type II FIPV.

### Cultivation of viruses:

Viruses were cultured in a fetal feline cell line, felis catus whole fetus (fcwf-4). A mixture consisting of equal amounts of Eagles minimum essential medium (E-MEM) and L-15 medium supplemented with 10% fetal calf serum (FCS) was used as the culture media.

### Purification of viruses:

225 cm² of fcwf-4 cells were inoculated with 100 TCID₅₀ of virus. After adsorption at 37°C for one hour, the culture media was added to the cells, and cultured in a CO₂ incubator. Cells showing CPE were removed with a cell scraper and harvested. The collected cells were washed three times with NTE buffer (10 mM Tris-HCl (pH7.0), 100 mM NaCl, and 2 mM EDTA), and then suspended in 5 mL of NTE buffer. After homogenating the suspended cells using a Dounce homogenizer, the resulting homogenates were centrifuged at 1,500 x g for ten minutes to remove the cell debris. They were then layered on top of 30% sucrose NTE buffer, and centrifuged at 200,000 x g for two hours to precipitate viral particles. The precipitate was dissolved in 0.5 mL of NTE buffer, then centrifuged at 15,000 x g for five minutes to obtain the supernatant as a viral solution.

### Protein fractionation:

Sample buffer (100 mM Tris-HCl (pH6.8), 4% SDS, 20% glycerol, and 0.1% BPB) was added to the purified virus, heated at 100°C for five minutes, and then electrophoresed (SDS-PAGE) on 11% polyacrylamide gel. The resultant gel was recovered, the position corresponding to the molecular weight of the N protein was determined from the position of a marker protein, and the section of the gel containing the N protein was cut out. The protein contained in the excised gel was recovered using Max Yield GP protein recovery system (ATTO, Tokyo) for use as the purified N protein. The purified N protein was used as an antigen for Western blotting, ELISA, intradermal test, and subunit vaccine.

### [2] The nucleotide sequence of type I FIPV strain KU-2

### Preparation of viral RNA:

After adding 0.5% SDS to the purified viruses of type I FIPV strain KU-2, the mixture was subjected to phenol extraction and ethanol precipitation to collect RNAs. The dried pellet was dissolved in RNase-free water to prepare an RNA solution.

### RT-PCR:

The nucleotide sequences of the primers used for cloning are shown below:
IMPr-3F (sense)
   5'-ggggaattcaattaaaggcaactactgcca-3' (SEQ ID NO: 3)
BEP(dT)₂₁ (antisense)
   5'-ctgtgaattctgcaggatccttttttttttttttttttttt-3' (SEQ ID NO: 4)

A restriction enzyme recognition sequence for cloning was added to each primer.

Minus strand primer, reverse transcriptase, and viral RNA were added to the reverse transcriptase reaction buffer, and reverse transcription was performed at 42°C for one hour, to produce the transcribed cDNAs. PCR primer, Taq polymerase, and the cDNAs were added to the PCR buffer, and the cDNAs were amplified by PCR under conditions of 95°C for five minutes, 30 cycles of (95°C for one minute, 55°C for one minute, and 72°C for two minutes), and 72°C for five minutes. The specificity of the PCR product was confirmed by 1% Agarose gel electrophoresis.

### Cloning:

The plasmid vector pUC18 was used for cloning. The PCR products were subjected to ethanol precipitation, dissolved in sterile distilled water, and then digested by adding a restriction enzyme and a ten-fold diluted buffer for restriction enzymes. pUC18 was also digested with the same restriction enzyme, and dephosphorylated using alkaline phosphatase. Both samples were subjected to 1% low-melting point agarose gel electrophoresis, and the part of the gel containing the DNA was cut out to extract and purify the DNA.

The purified PCR products and the DNA fragments of the vector were mixed in a ligation buffer, DNA ligase was added to the mixture, and a ligation reaction was then performed at 15°C for one hour. Circularized DNA was transformed into competent *E. coli* JM109 strain cells. The resulting cells were plated onto ampicillin-supplemented agar plates, and then cultured overnight at 37°C.

Colonies formed on the agar plate were picked, and then cultured overnight in 1.5 mL of ampicillin-supplemented L-Broth. Plasmids were extracted from the cells and cleaved by a restriction enzyme. The size of the resulting DNA fragments was confirmed by 1% agarose gel electrophoresis to determine whether cloning was successful or not. *E. coli* confirmed to be cloned was then cultured overnight in 25 mL of ampicillin-supplemented L-Broth to extract and purify the recombinant plasmid DNAs.

### Sequencing:

Cloned cDNAs were further subcloned into M13mp18/19 phage vectors. Single-stranded DNAs were purified from the resulting phages and used for sequencing. The nucleotide sequences were analyzed using an autosequencer, based on the dideoxynucleotide chain termination method.

### Nucleotide sequences:

Of the full length of the genetic RNA of FIPV, which is approximately 20 kb, the present inventors analyzed the 9.2 kb nucleotide sequence of the 3'-end of the KU-2 strain. Fig. 1 shows the cDNA nucleotide sequence of the N protein of the KU-2 strain, and the predicted amino acid sequence thereof. The N gene consists of the 1,131-nucleotide ORF from the initiation codon ATG to the stop codon TAA, and encodes 377 amino acids. The gene was predicted to express an early protein calculated to be 42.5 kDa.

### Homology:

Fig. 2 shows the amino acid sequence alignment of the N genes derived from, respectively, the type I FIPV strains KU-2, Black, and UCD1, type II FIPV strain 79-1146, type II feline enteric coronavirus (FECV) strain 79-1683, canine coronavirus (CCV) strain Insvc1, and swine transmissible gastroenteritis virus (TGEV) strain Purde. Comparison of these amino acid sequences showed that the KU-2 strain gene comprises many characteristic amino acid mutations that are not present in the other six strains, that is, the KU-2 strain has an unique sequence.

Table 1 shows the amino acid sequence homology and nucleotide sequence homology of the N proteins. The homologies in the table were calculated using Maximum Matching from the genetic information processing software, GENETYX. The parameters are shown above (Takashi K. and Gotho O. (1984) J. Biochem. 92:1173-1177). Matching condition: matches= -1, mismatches= 1, gaps= 1, *N+= 2

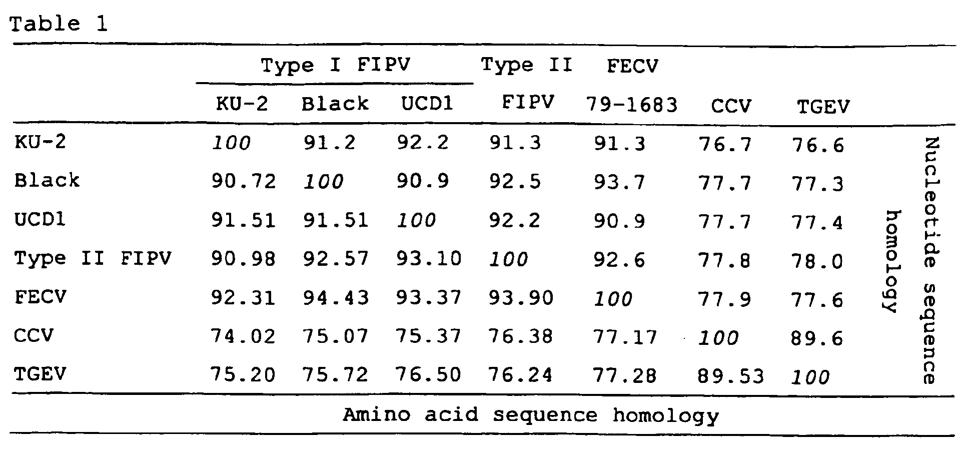

The names of the viruses in the table each indicate the viruses below. The numerical values in the upper right of the 100% diagonal show nucleotide sequence homology, and those to the lower left show amino acid sequence homology.
KU-2: Type I FIPV strain KU-2 (GenBank Accession No. AB086881)
Black: Type I FIPV strain Black (GenBank Accession No. AB086903)
UCD1: Type I FIPV strain UCD1 (GenBank Accession No. AB086902)
Type II FIPV: Type II FIPV strain 79-1146 (GenBank Accession No. X56496)
FECV: Feline enteric coronavirus strain 79-1683 (GenBank Accession No. AB086904)
CCV: Canine coronavirus CCV strain Insavc-1 (GenBank Accession No. D13096)
TGEV: Transmissible gastroenteritis virus strain Purdue
(GenBank Accession Nos. M21627 and M14878)

A comparison of the amino acid sequences of the N protein of the KU-2 strain with those of feline coronaviruses, type I and type II FIPV, and type II FECV showed approximately 90% homology, and approximately 75% homology with those of CCV and TGEV. In addition, each comparison between feline coronaviruses showed about 90% homology, revealing that each of these viruses comprise a unique genetic sequence, even though most regions of the genes are conserved.

### Phylogenetic tree:

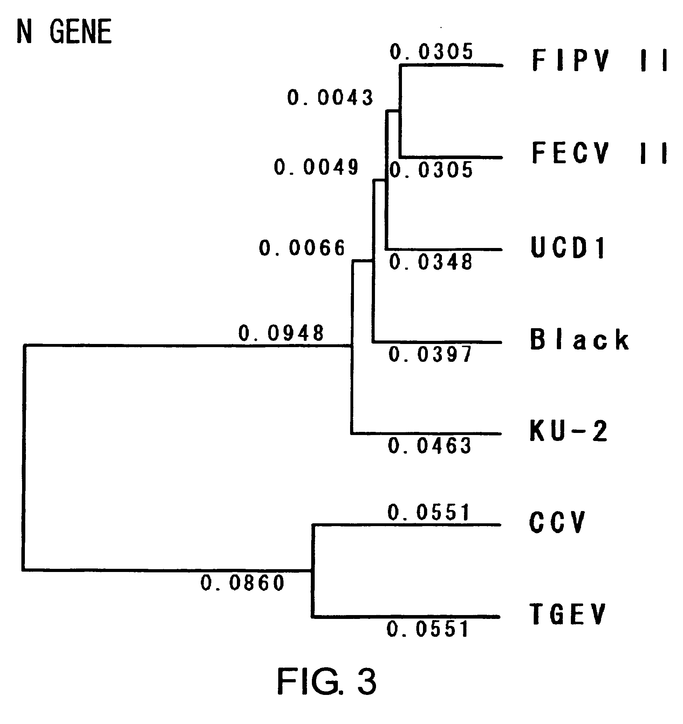

Fig. 3 shows the phylogenetic tree prepared from the amino acid sequences of the N genes.

In the phylogenetic tree analysis of the N genes, feline coronaviruses, type I FIPV, type II FIPV, and FECV form a single group. This group was shown to be distal from canine (CCV) and porcine (TGEV) coronaviruses. Although the difference was only slight, the N gene of the KU-2 strain was the most evolutionarily distant of the feline coronaviruses.

### [3] Expression of recombinant N proteins

### RT-PCR:

Based on the nucleotide sequence of the FIPV strain KU-2, PCR primers were constructed upstream and downstream of the N protein open reading frame (ORF) so that the entire N protein could be expressed. Restriction enzyme recognition sequences (BamHI and SalI) for cloning were added to the primers.

Using viral RNA prepared from the purified FIPV strain KU-2 as a template, a reverse transcription reaction was performed using oligo dT primers to synthesize cDNAs. cDNAs were then amplified by PCR under conditions of 95°C for five minutes, 30 cycles of (95°C for one minute, 55°C for one minute, and 72°C for two minutes), and 72°C for five minutes. The specificity of the PCR products was confirmed by 1% agarose gel electrophoresis.

### Cloning:

Cloning into the pFastBac1 plasmid vectors was performed as for cloning into the pUC18 vectors. BamHI and SalI were used as the restriction enzymes for recombination. *E. coli* HB101 strain was used as the host. *E. coli* confirmed to have been cloned was then cultured overnight in 25 mL of ampicillin-supplemented L-Broth. Recombinant pFastBac1 plasmid DNAs were extracted from the culture and purified.

### Preparation of recombinant baculoviruses:

Purified plasmid DNAs were transfected into competent *E. coli* DH10BAC strain cells. The resulting cells were plated onto agar plates supplemented with kanamycin, tetracycline, gentamicin, IPTG, and Bluo-gal, and then cultured overnight at 37°C. *E. coli* DH10BAC strains contain the baculovirus shuttle vector, bMON14272, and thus DH10BAC cells transfected with recombinant pFastBac1 can recombine the N protein genes to bMON14272. White colonies were selected from the colonies that formed on the agar plate, and were cultured overnight in 25 mL of L-Broth supplemented with kanamycin, tetracycline, and gentamicin, to extract and purify recombinant bMON14272 DNA.

The purified recombinant bMON14272 DNAs were mixed with CELLFECTIN, and transfected into *Spodoptera frugiperda*-derived cells (SF-9). After transfection, the mixture was removed, and the cells were cultured for two days in 10% FCS-supplemented TC-100 medium (Insect Medium). Baculovirus produced in the culture supernatant was collected to use as recombinant baculovirus in expression experiments.

### Expression of recombinant proteins:

SF-9 cells were seeded into a culture flask (225 cm²) at 1x 10⁶ to 2x 10⁶ cell/mL, and cultured for two days. The culture medium was removed, and then recombinant baculovirus was inoculated to the cells. After adsorption at 27 °C for one hour, FBS-free TC-100 media was added to the cells and cultured for 96 hours. Resulting infected cells were harvested and washed with phosphate buffered saline (PBS⁽⁻⁾). 4 mL of 0.2% NP-40-supplemented RSB solution (0.01 M NaCl, 0.0015 M MgCl₂, and 0.01 M Tris-HCl (pH7.4)) was added to the cells for lysing. This was then centrifuged at 5,000 rpm for ten minutes. The precipitates were suspended in 1 mL PBS⁽⁻⁾ to use as recombinant N protein in experiments.

### [4] Confirmation of specificity

### Western blotting:

Sample buffer was added to the prepared antigens, heated at 100°C for five minutes, and then subjected to SDS-PAGE on an 11% gel. The gel was then recovered to transfer onto a PVDF membrane using a semidry-type blotting apparatus. The transferred membrane was washed with PBS⁽⁻⁾, and then blocked by incubating overnight in BlockAce at 4°C.

A mixture of monoclonal antibodies against the FIPV N protein (Clone No. E22-2), monoclonal antibodies against the FIPV M protein (Clone No. F18-2), and monoclonal antibodies against the FIPV S protein (Clone No. 6-4-2) was used as the primary antibody. The transferred membrane was reacted in the monoclonal antibody mixture at 37 °C for two hours, and then washed three times with 0.05% Tween20 PBS⁽⁻⁾. These monoclonal antibodies are already known, as shown below.

### Clone No. E22-2 and Clone No. F18-2:

(Hohdatsu T, Sasamoto T, Okada S, Koyama H. Antigenic analysis of feline coronaviruses with monoclonal antibodies (MAbs): preparation of MAbs which discriminate between FIPV strain 79-1146 and FECV strain 79-1683.Vet Microbiol. 1991 Jun;28(1):13-24)

### Clone No. 6-4-2:

(Hohdatsu T, Okada S, Koyama H. Characterization of monoclonal antibodies against feline infectious peritonitis virus type II and antigenic relationship between feline, porcine, and canine coronaviruses. Arch Virol. 1991;117(1-2):85-95)

HRPO-labeled rabbit anti-mouse IgG + M + A antibody was used as the secondary antibody. The membrane was soaked in antibody diluent supplemented with the labeled antibodies, reacted at 37°C for one hour, and then washed three times with 0.05% Tween20 PBS⁽⁻⁾. The transferred membrane was stained by soaking in a substrate solution (0.02% DAB, 0.006% H₂O₂, and 0.05 M Tris-HCl (pH7.6)) for approximately five to 20 minutes. Then, the reaction was stopped by washing the membrane with distilled water.

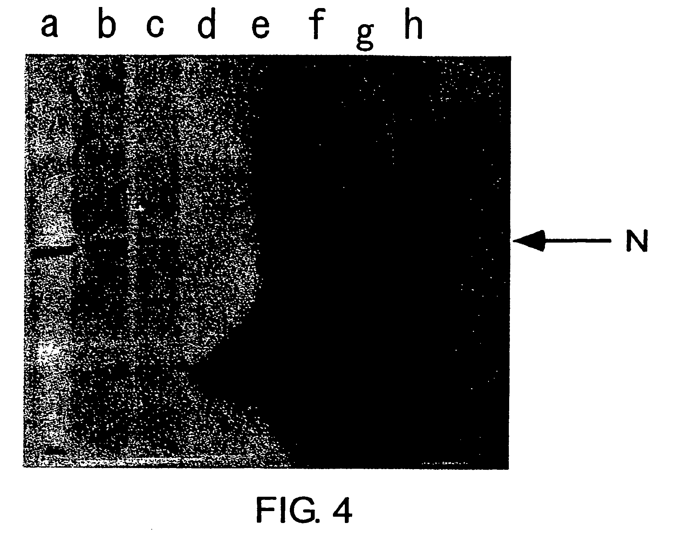

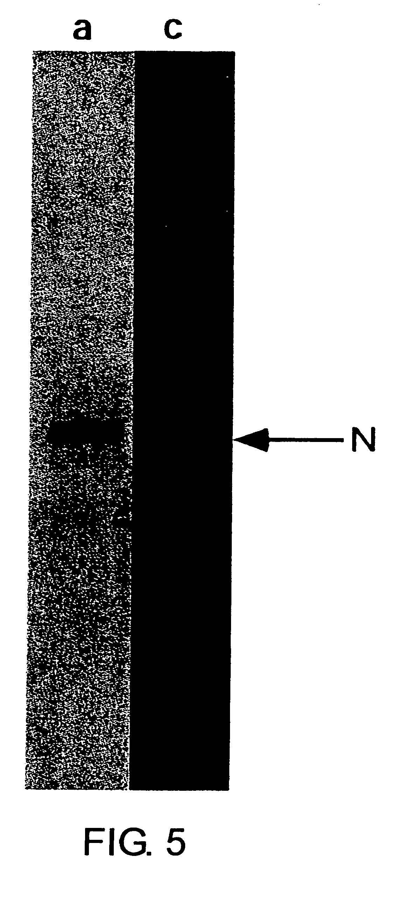

The N protein of the FIPV strain KU-2 was estimated, according to calculations based on its amino acid sequence, to be an approximately 45-kDa expression product. In fact, the recombinant protein expressed in insect cells was detected as a specific band at a position of 40- to 45-kDa, and was confirmed to be the recombinant FIPV N protein (Fig. 4). Purified N protein also showed a specific band at the same position (Fig. 5).

### ELISA:

The prepared antigen was diluted two to 128 times by two-fold serial dilutions using a coating buffer (0.1 M carbonate buffer), then aliquoted into an ELISA plate, and immobilized overnight at 4°C. Each well was washed three times with 0.05% Tween20 PBS⁽⁻⁾, and then used for ELISA.

The culture supernatant of anti-FIPV N protein monoclonal antibody (Clone No. E22-2) was used as the primary antibody. 0.1 mL/well of culture supernatant was aliquoted into the antigen-immobilized well. After reacting at 37°C for one hour, each well was washed three times with 0.05% Tween20 PBS⁽⁻⁾. HRPO-labeled rabbit anti-mouse IgG + M + A antibody was used as the secondary antibody. The reaction was performed at 37°C for one hour in antibody diluent supplemented with the labeled antibody, and then each well was washed three times with 0.05% Tween20 PBS⁽⁻⁾. TMB substrate solution (tetramethylbenzidine) was aliquoted at 0.1 mL/well, then reacted at room temperature for 20 minutes. 0.05 mL of H₂SO₄ was added per well, and optical density was measured at 450 nm.

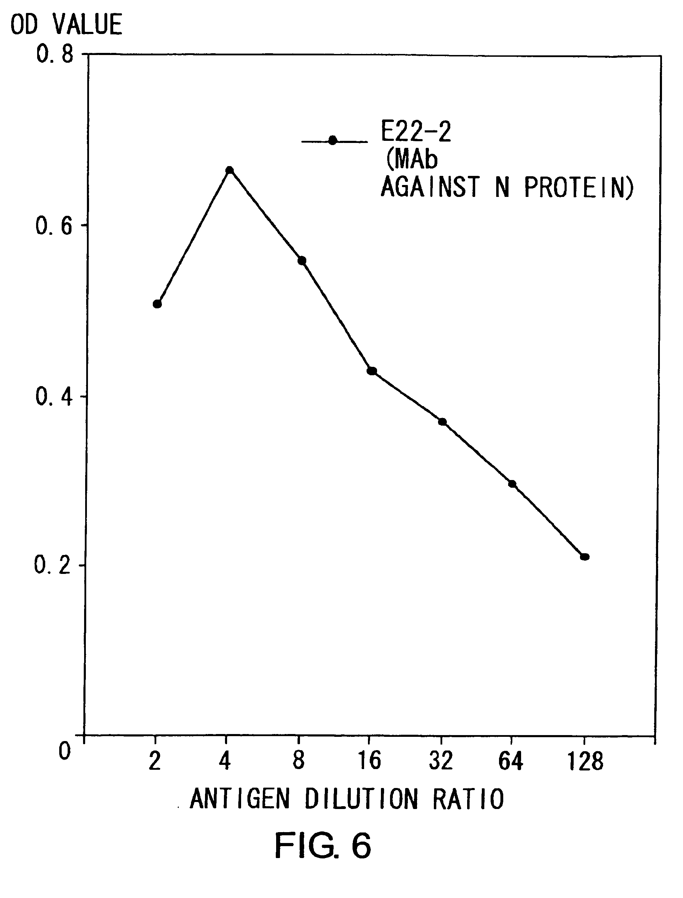

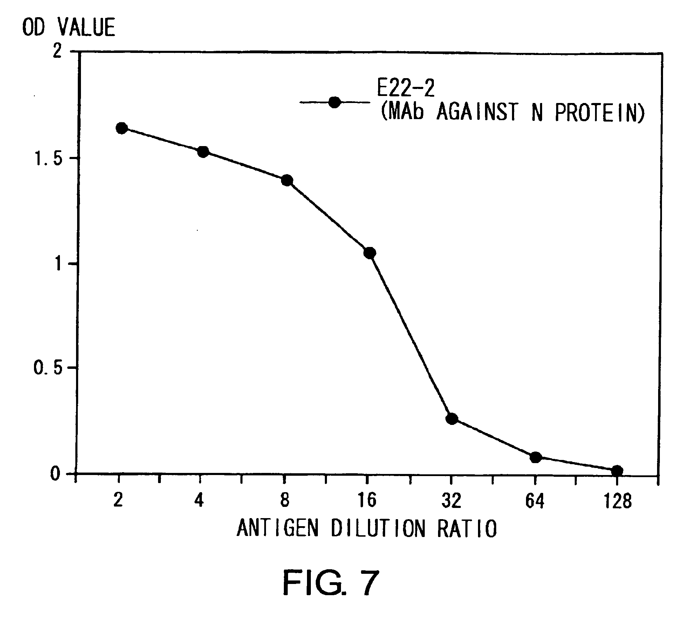

Both recombinant N protein (Fig. 6) and purified N protein (Fig. 7) reacted against the anti-FIPV N protein monoclonal antibody in an antigen amount-dependent manner, confirming their specificity.

### [5] Vaccine production

### Antigen preparation:

The amount of antigen was determined by Western blotting. Prepared antigen was diluted eight to 512 times by two-fold serial dilutions, then subjected to SDS-PAGE, and was detected by Western blotting using anti-FIPV N protein monoclonal antibody (Clone No. E22-2). An antigen amount of one unit was defined as the amount in the detectable lane with the highest dilution ratio, and the reciprocal of this dilution ratio was defined as the amount of antigen in the stock solution. The vaccine was prepared by dilution with PBS⁽⁻⁾ such that a single dose contained 16 units of antigen.

### Adjuvant:

Felidovac PCR (InterVet), a feline inactivated trivalent vaccine that is commercially available in Japan, was used as the adjuvant. This vaccine contains Adjuvant L80 and aluminum hydroxide. 1 mL of antigen adjusted to 16 units/mL and 1 mL of Felidovac PCR (one dose) were combined and mixed well to produce the vaccine. 2 mL of the resulting vaccine was used in a single dose.

### [6] Immunization test (1)

### Animals:

Seven- to nine-month old SPF cats were used for the experiments. Four cats were used in the group immunized with the recombinant N protein vaccine. Four animals were used in the challenge control group, and were immunized with antigens obtained by treating SF-9 cells using a method similar to that for collecting recombinant N proteins.

### Vaccination:

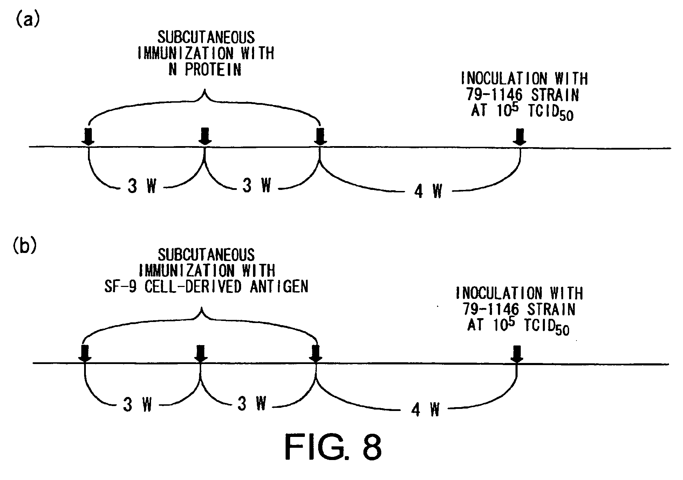

A single vaccine dose was administered subcutaneously to the neck, three times at three-week intervals (Fig. 8). Blood was collected immediately before each immunization, and the antibody titer of the serum was measured.

### Antibody titer measurement by ELISA:

Purified N protein was diluted with coating buffer such that two units (approximately 100 ng/well) were aliquoted to an ELISA plate. Immobilization was carried out overnight at 4°C. Each well was washed, and then the primary serum, which was cat serum collected and then diluted 100 times with an antibody diluent, was added to the well. This was reacted at 37°C for one hour. After washing each well, HRPO-conjugated anti-cat Ig was added thereto, and reacted at 37°C for one hour. After washing, substrate solution (tetramethylbenzidine) was added to each well. Coloring was performed at room temperature for 20 minutes. A quenching solution was added to each well, and then optical density at 450 nm was measured.

Three weeks after the second immunization (at the time of the third immunization), none of the cats in the group immunized with recombinant N protein vaccine showed an increase in antibody titer. However, four weeks after the third immunization (at the time of challenge), their antibody titer increased. Of these cats, Cat No. 221 showed a remarkable increase in antibody titer, although the other three cats showed only weak reactions. In the control group, which was immunized with an antigen prepared from SF-9 cells, an increase in ELISA OD values was not observed in any of the four animals (Fig. 9).

### Measurement of neutralizing antibody titer:

0.025 mL of cat serum was subjected to two-fold serial dilutions, and then mixed on a 96-well plate with an equal amount of a viral solution prepared to be 200 TCID₅₀/0.025 mL. This was then reacted at 4°C for 24 hours. fcwf-4 cells were added to the mixture at 1x 10⁶ cells/well, and were cultured for 48 hours. The reciprocal of the highest cat serum dilution ratio that completely suppressed CPE (cytopathogenic effect) was taken to be the neutralizing antibody titer.

Since anti-N protein antibodies do not normally have neutralizing activity, it may be difficult to suppress CPE by the sole use of antibodies produced by N protein immunization. Thus, a neutralization test was carried out with the addition of a complement. More specifically, a viral solution prepared to be 200 TCID₅₀/0.025 mL was supplemented with 10% rabbit serum (complement) to use for reaction in the same way.

Regardless of the presence of the complement, the neutralizing antibody titer in all cats in the recombinant N protein-immunized group was less than ten-fold at the time of immunization initiation, as well as four weeks after the third immunization (the 70th day). The SF-9 cell-derived antigen-immunized group showed the same result (Table 2).

**Table 2**

| | | Neutralizing antibody titer | | | |
|---|---|---|---|---|---|
| | | With complement | | Without complement | |
| Groups | Cat No. | Antigen inoculation | 70 days after inoculation | Antigen inoculation | 70 days after inoculation |
| Baculovirus recombinant N protein-immunized group | 217 | <10 | <10 | <10 | <10 |
| | 221 | <10 | <10 | <10 | <10 |
| | 191 | <10 | <10 | <10 | <10 |
| | 6 | <10 | <10 | <10 | <10 |
| SF-9-immunized group | 163 | <10 | <10 | <10 | <10 |
| | 210 | <10 | <10 | <10 | <10 |
| | 170 | <10 | <10 | <10 | <10 |
| | 173 | <10 | <10 | <10 | <10 |

### Measurement of cellular immunity:

In order to determine the degree of cellular immunity conferred by the vaccine, delayed-type hypersensitivity to the purified N protein antigen was measured as an intradermal reaction.

The left flanks of the cats were shaved and disinfected with 70% ethanol, and then 0.1 mL each of N protein antigen (0.1 mg/mL) and the control, PBS⁽⁻⁾, were injected intradermally. Antigen injection sites were separated by approximately 4 cm. 24, 48, 72, and 96 hours after injection, the extent of swelling that appeared at the injection site was measured using calipers.

A response to the purified N protein was observed in all cats in the group immunized with recombinant N protein. Swelling in Cat No. 217 was observed 24 hours after injection. This response decreased and then disappeared 96 hours later. Similarly, in Cat Nos. 191 and 221, swelling was observed after 24 hours, and then this response decreased; however, 2 to 3 mm of swelling was observed even after 96 hours. Swelling in Cat No. 6 could be observed after 72 hours, but the response was weak since the maximum size was 2 mm, and the response had disappeared after 96 hours (Fig. 10). None of the cats showed swelling for PBS⁽⁻⁾. These results revealed that the recombinant N protein vaccine confers cellular immunity to the cats.

### [7] Challenge test (1)

### Challenge method:

On the fourth week after the third immunization, a challenge test was performed on those cats subjected to immunization test (1) (Fig. 8).

Type II FIPV strain 79-1146 was used as the challenging virus, and 10⁵ TCID₅₀ (1 mL) of the virus was inoculated orally and intranasally. The clinical symptoms were observed daily, from the day of viral inoculation to the completion of the experiment. Body temperature and body weight were measured every three days. Rectal swab was also collected every three days for use in later experiments. Blood was collected every six days, and serum was separated for use in later experiments.

### Survival rate:

All four of the challenge control group cats, Nos. 170, 173, 210, and 163, developed FIP and died on the 23rd, 26th, 31st, and 44th day after challenge, respectively. On the other hand, although Cat No. 191 developed FIP and died on the 48th day, infection was confirmed in the remaining three cats of the recombinant N protein-immunized group, but development of the disease was prevented, and thus the cats survived (Fig. 11).

### Changes in body temperature:

In the recombinant N protein-immunized group, fever was commonly observed to develop immediately after challenge, however, the clinical course thereafter differed depending on the individual. Cat No. 217 developed a fever of more than 40°C on the third day after inoculation, but had normal temperature thereafter. Cat No. 221 developed a fever of close to or more than 40°C on the third day, and from the 27th to the 42nd day after inoculation, but its temperature decreased thereafter. Cat No. 191 developed a fever of more than 40°C on the third day, and from the 27th to the 33rd day, and then died on the 48th day after a sudden drop in body temperature. Cat No. 6 developed a fever of more than 40°C on the third day, and after that its temperature continued to be normal.

On the other hand, in the challenge control group, all four animals developed a bimodal fever. Cat No. 163 developed a fever close to or greater than 40°C on the third day, from the 18th to 21st day, and from the 30th to the 36th day, and died on the 44th day after a sudden drop in body temperature. Cat No. 210 developed a fever of more than 40°C on the third day, and from the 12th to the 27th day, and died on the 31st day. Cat No. 170 developed a fever close to or greater than 40°C on the third day, and from the 15th to the 18th day, and died on the 23rd day after a sudden drop in body temperature. Cat No. 173 developed a fever of more than 40°C on the third day, and from the 18th to the 21st day, and died on the 26th day after a sudden drop in body temperature.

### Changes in body weight:

In the recombinant N protein-immunized group, the body weight of the two cats, Nos. 217 and 6, increased smoothly. A slight decrease was observed in the body weight of Cat No. 221, but its weight was virtually maintained at a constant level. Although the decrease was not sharp, the body weight of Cat No. 191 decreased gradually, and the cat died on the 48th day. In the challenge control group, the body weight of all four animals began to be drastically reduced after viral challenge, and continued to decrease until they died.

### ELISA antibody titer:

Antibody titer in the serum was measured every six days after challenge by ELISA, using purified N protein as the antigen (Fig. 12).

Of the four animals in the recombinant N protein-immunized group, Cat No. 221 reacted most to vaccination. Its antibody titer started to increase immediately after challenge and reached a plateau at around the 18th day. The remaining three animals had low reactions to vaccination. Their antibody titer began to increase from the sixth day after challenge, reaching a virtual plateau at around the 18th day. The antibody titer in all animals of the challenge control group began to increase on the sixth day after challenge, but the reaction was not as rapid as in the recombinant N protein-immunized group, and the antibody titer did not increase much on the 12th day. The antibody titer in all cats continued to increase until their death.

### Neutralizing antibody titer:

A neutralization test (without using complements) was performed on sera obtained at the time of challenge, on the 12th and 60th day after challenge, and at the time of death (Table 3).

**Table 3**

| | | Neutralizing antibody titer | | | |
|---|---|---|---|---|---|
| Groups | Cat No. | 79-1196 strain inoculation | 12 days after 79-1146 strain inoculation | 60 days after 79-1146 strain inoculation* | Survival |
| Baculovirus recombinant N protein-immunized group | 217 | <10 | 20 | 6400 | Survived |
| | 221 | <10 | 160 | 6400 | Survived |
| | 191 | <10 | 40 | 6400 | Died |
| | 6 | <10 | 40 | 1600 | Survived |
| SF-9 cell-derived antigen-immunized group | 163 | <10 | 80 | 6400 | Died |
| | 210 | <10 | 80 | 1600 | Died |
| | 170 | <10 | 80 | 800 | Died |
| | 173 | <10 | 80 | 1600 | Died |

| | | | | | |
|---|---|---|---|---|---|
| *For cats that died prior to 60 days after inoculation, the titer at the time of death is shown. | | | | | |

At the time of challenge, the neutralizing antibody titer of the recombinant N protein-immunized group was ten-fold or less in all four cats. Twelve days after challenge, the neutralizing antibody titer of Cat No. 221, which responded strongly to ELISA, had increased by 160-fold, but the other three animals showed a 20-to 40-fold increase. The neutralizing antibody titer on the 60th day after challenge, or at death, increased by 1600-fold in one surviving animal and 6400-fold in the other two surviving animals, and 6400-fold in the one animal that died. The neutralizing antibody titer of the challenge control group was ten-fold or less in all four animals at the time of challenge, 80-fold in all four animals on the 12th day after challenge, and 800- to 6400-fold at the time of death. Accordingly, for both the recombinant N protein-immunized group and the challenge control group, neutralizing antibody titer and clinical course (survival) showed no correlation.

### Virus isolation:

RNAs were extracted from the rectal swab collected every three days after challenge. Viral growth was confirmed in the body by detection of the viral gene using RT-nested PCR (Table 4).

**Table 4**

| | | Days after 79-1146 strain inoculation | | | | | | |
|---|---|---|---|---|---|---|---|---|
| Groups | Cat No. | 0 | 3 | 6 | 9 | 12 | 15 | 18 |
| Baculovirus recombinant N protein-immunized group | 217 | - | - | - | - | - | - | - |
| | 221 | - | - | - | - | - | - | - |
| | 191 | - | - | - | - | - | - | + |
| | 6 | - | | - | - | - | - | + |
| SF-9 cell-derived antigen-immunized group | 163 | - | - | - | - | + | + | - |
| | 210 | - | - | - | - | - | - | - |
| | 170 | - | + | | + | + | + | + |
| | 173 | - | - | - | - | - | - | - |

A commercially available Sepa Gene RV-R kit (Sanko Junyaku, Tokyo) was used for the viral RNA extraction. The extracted RNAs were dissolved in 0. 007 mL water to prepare a purified RNA solution. The purified RNA solution was heated at 80°C for five minutes and cooled rapidly to cause thermal denaturation. The resulting solution was mixed with reverse transcriptase buffer, primers, and reverse transcriptase, and then reacted at 42°C for one hour to synthesize cDNAs. Then, as a template, 1 µL of cDNA obtained by reverse transcription reaction was added to a reaction solution of the following composition, and the total volume was adjusted to 50 µL using 36 µL of sterile distilled water:
4.8 µL of 10x reaction buffer;
5 µL of each 2.5 mM dNTP;
1 µL of 50 µM primer mix (outer primer set consisting of outer(+) and outer(-)); and
2.2 µL of Taq polymerase (1 unit/2.2 µL).

The above-described mixture was placed into a DNA thermal cycler to amplify the DNAs by PCR. The reaction was an initial thermal denaturation at 94°C for 3 minutes, 30 cycles of 94°C for one minute (thermal denaturation), 55°C for one minute (annealing), and 72°C for two minutes (elongation reaction), and a final elongation reaction at 72°C for five minutes.

Using 1 µL of PCR product amplified using the outer primer set, PCR was similarly performed with the inner primer set (inner (+) and inner(-)). The primers used for the PCR specifically recognize the N gene of FCoV. The nucleotide sequences of each of the primers are shown below:
outer(+): 5'-CAACTGGGGAGATGAACCTT-3' (SEQ ID NO: 5);
outer(-): 5'-GGTAGCATTTGGCAGCGTTA-3' (SEQ ID NO: 6);
inner(+): 5'-ATTGATGGAGTCTTCTGGGTTG-3' (SEQ ID NO: 7); and
inner(-): 5'-TTGGCATTCTTAGGTGTTGTGTC-3' (SEQ ID NO: 8).

PCR primers, Taq polymerase, and cDNA were added to a PCR buffer to perform 30 cycles of PCR. The resulting PCR products were confirmed using 1% Agarose gel electrophoresis, and those for which a band was detected at a specific position were determined to be positive.

The virus was detected in two animals in the recombinant N protein-immunized group. RNA was detected in Cat No. 191 on the 18th day after challenge, and in Cat No. 6 on both the third and 18th days. The virus was detected in three animals in the challenge control group. The virus was detected in Cat No. 163 on the 12th and 15th days, in Cat No. 170 on the third day, and from the ninth to the 18th days, and in Cat No. 173 on the third day. The virus was detected in Cat Nos. 6, 170, and 173 on the third day of challenge, and this result coincided with the period of fever development immediately after challenge. Virus isolation results showed no correlation with survival.

### [8] Immunization test (2)

### Animals:

Using six-month old SPF cats, an immunization test was carried out as for immunization test (1).

Four animals were used for the recombinant N protein-immunized group, four animals were used for immunization with type II FIPV strain KU-1-derived purified N protein vaccine, and four animals were used for the challenge control group.

### Vaccination:

The vaccine was administered as in immunization test (1) (Fig. 13).

### Antibody titer measurement by ELISA:

In the recombinant N protein-immunized group, several animals showed a slight response three weeks after the second immunization, and a clear increase in response was observed in all four animals four weeks after the third immunization (Fig. 14). In the purified N protein-immunized group also, several animals showed a slight response three weeks after the second immunization, and a clear increase in response was observed in all four animals four weeks after the third immunization, the values of which were higher than for the recombinant N protein-immunized group. On the other hand, none of the four animals in the control group showed a response.

### Measurement of neutralizing antibody titer:

Neutralizing antibodies were measured in sera collected before immunization, and on the fourth week after the third immunization (before challenge), using only methods that did not use complements.

In all animals in all groups, that is, in the recombinant N protein-immunized group, the purified N protein-immunized group, and the control group, the increase pre- and post-immunization was of ten-fold or less, and neutralizing antibodies could not be detected (Table 5).

**Table 5**

| | | Neutralizing antibody titer | | | | |
|---|---|---|---|---|---|---|
| Groups | Cat No. | Immunization initiation | 79-1146 strain inoculation | 12 days after 79-1146 strain inoculation | 60 days after 79-1146 strain inoculation* | Survival |
| Baculovirus recombinant N protein-immunized group | 177 | <10 | <10 | 40 | 200 | Died |
| | 242 | <10 | <10 | 20 | >6400 | Survived |
| | 245 | <10 | <10 | 20 | 3200 | Survived |
| | 247 | <10 | <10 | 10 | 3200 | Survived |
| Purified FIPV N protein-immunized group | 175 | <10 | <10 | 20 | >6400 | Died |
| | 180 | <10 | <10 | 40 | 80 | Died |
| | 243 | <10 | <10 | 10 | >6400 | Survived |
| | 252 | <10 | <10 | 40 | >6400 | Died |
| SF-9 cell-derived antigen-immunized group | 178 | <10 | <10 | 10 | 400 | Died |
| | 181 | <10 | <10 | 40 | 3200 | Died |
| | 244 | <10 | <10 | 40 | 6400 | Survived |
| | 249 | <10 | <10 | 40 | 200 | Died |

| | | | | | | |
|---|---|---|---|---|---|---|
| *For cats that died prior to 60 days after inoculation, the titer at the time of death is indicated. | | | | | | |

### Measurement of cellular immunity:

As well as immunization test (1), cellular immunity was measured as delayed-type hypersensitivity to the purified N protein. The results are shown in Fig. 15 as the average value from four animals.

Swelling was observed in the recombinant N protein-immunized group from 24 hours after inoculation, peaked at this time, and then gradually disappeared. In the purified N protein-immunized group, swelling was observed from 24 hours after inoculation, reached a maximum 48 hours after inoculation, and then decreased, however about 1 mm of swelling was observed even after 96 hours.

### [9] Challenge test (2)

### Challenge method:

The cats subjected to immunization test (2) were challenged with FIPV 79-1146 by the same method as in challenge test (1) (Fig. 13). Observation of clinical symptoms, measurement of body temperature and body weight, blood collection, and rectal swab collection were performed as in challenge test (1). In addition, laryngeal swab were collected every three days and used for virus isolation.

### Survival rate:

In the challenge control group, Cat Nos. 249, 178, and 181 died on the 23rd, 29th, and 60th day after challenge, respectively, but Cat No. 244 survived. Thus, three out of the four animals died (Fig. 16). In the recombinant N protein-immunized group, however, three animals survived, and only Cat No. 177 died on the 19th day. In the purified N protein-immunized group, Cat Nos. 180, 175, and 252 died on the 19th, 77th, and 78th day, respectively. Thus, three out of the four animals died. However, a survival advantage was observed compared to the challenge control group, and an effect was confirmed to a certain degree.

### Changes in body temperature:

In the recombinant N protein-immunized group, Cat No. 177 showed a continued increase in body temperature after challenge. Its temperature peaked on the 15th day, suddenly dropped, and then the cat died. Cat No. 242 developed a fever on the third day. Its body temperature decreased once, but on about day 30 the fever redeveloped and continued for some time. The other two animals developed fever in the early stages of infection, but virtually normal temperatures continued thereafter.

In the purified N protein-immunized group, Cat No. 180 developed a fever of over 40°C on the third and 12th days after challenge, then showed a sudden drop in body temperature, and died. The body temperature of Cat No. 252 continued to be high, at around 40°C, and then dropped gradually, leading to death. Cat No. 175 maintained near-normal temperature after developing a fever in the initial stages of infection, but redeveloped a high fever on the 72nd day, and died on the 77th day. Cat No. 243 maintained nearly normal temperature. In the challenge control group, Cat No. 244 stayed at around normal temperatures. In the three animals that died, after developing fever in the early stages of infection, the temperature decreased once at around the 12th day, but fever soon redeveloped and this continued until a few days before death, when body temperature dropped.

### Changes in body weight:

In the recombinant N protein-immunized group, Cat No. 177, which died, showed a continuous decrease in body weight from immediately after infection. The body weight of Cat Nos. 245 and 247 smoothly increased, while the body weight of Cat No. 242 gradually decreased. In the purified N protein-immunized group, Cats No. 180 and No. 252 showed a sudden decrease and gradual decrease in body weight, respectively, then both died. Cat No. 175 showed an increase in body weight, but died at around the same time as Cat No. 252. Cat No. 243 showed a smooth increase in body weight. In the challenge control group, Cat No. 244, which survived, showed a slight increase in body weight, while the body weight of the three animals that died continued to decrease from immediately after infection until the time of death.

### ELISA antibody titers:

The antibody titers in serum were measured every six days after challenge by ELISA using purified N protein (Fig. 17). There are no fundamental differences between the recombinant N protein-immunized group and the purified N protein-immunized group. Specifically, the number of antibodies increased six days after challenge, and reached a plateau twelve days after challenge. In the purified N protein-immunized group, the titer of Cat No. 180 was already high at the time of challenge, and further increased after challenge. In the challenge control group, responses were observed from the 12th day after challenge in all four animals, and reached a plateau around the 30th day.

### Neutralizing antibody titer:

No fundamental differences were observed regarding changes in neutralizing antibody titer for the recombinant N protein-immunized group, purified N protein-immunized group, and challenge control group (Table 5). The antibody titers in all cats before challenge were ten-fold or less. On the 12th day after challenge, the antibody titers showed a ten- to 40-fold increase. The titers of those cats alive on the 60th day after infection showed a 3200- to 6400-fold increase or more. Those cats that died prior to 60th day showed a low increase of 80- to 200-fold, suggesting the cats may have died before sufficient antibodies were produced.

### Virus isolation using fcwf-4 cells:

0.05 mL/well of laryngeal swabs and rectal swabs were individually inoculated into fcwf-4 cells cultured in a 48-well plate, and adsorption was carried out at 37°C for one hour. Cell surfaces were washed with the culture media. 0.5 mL/well of MEM maintenance medium was added to the plate and cultured at 37°C. Those in which CPE was observed within two days were determined to be positive. These results were virtually the same as those of RT-PCR. Virus isolation from rectal swabs showed that two animals from the recombinant N protein-immunized group and one animal from the purified N protein-immunized group were positive for between one and two days from the sixth to the 15th day after challenge (Table 6).

**Table 6**

| | Groups | Cat No. | Days after FIPV 79-1146 strain inoculation (days) | | | | | |
|---|---|---|---|---|---|---|---|---|
| | | | 0 | 3 | 6 | 9 | 12 | 15 |
| Virus isolation using fcwf-4 | Baculovirus recombinant N protein-immunized group | 177 | - | - | - | - | + | + |
| | | 242 | - | - | - | - | - | - |
| | | 245 | - | - | - | - | - | - |
| | | 247 | - | - | - | - | + | - |
| | Purified FIPV N protein-immunized group | 175 | - | - | - | - | - | - |
| | | 180 | - | - | + | - | + | - |
| | | 243 | - | - | - | - | - | - |
| | | 252 | - | - | - | - | - | - |
| | SF-9 cell-derived antigen-immunized group | 178 | - | - | - | - | - | - |
| | | 181 | - | - | - | - | - | - |
| | | 244 | - | - | - | - | - | - |
| | | 249 | - | - | - | - | - | - |
| Detection of FCoV gene using RT-nPCR | Baculovirus recombinant N protein-immunized group | 177 | - | - | - | - | + | + |
| | | 242 | - | - | - | - | - | - |
| | | 245 | - | - | - | + | + | - |
| | | 247 | - | - | - | - | + | - |
| | Purified FIPV N protein-immunized group | 175 | - | - | - | + | - | - |
| | | 180 | - | - | + | - | + | - |
| | | 243 | - | - | - | - | - | - |
| | | 252 | - | - | - | - | - | - |
| | SF-9 cell-derived antigen-immunized group | 178 | - | - | - | + | - | - |
| | | 181 | - | - | - | - | - | + |
| | | 244 | - | - | + | - | - | - |
| | | 249 | - | - | - | - | - | - |

| | | | | | | | | |
|---|---|---|---|---|---|---|---|---|
| +: Virus was isolated, or FCoV gene was detected -: Virus was not isolated, or FCoV gene was detected | | | | | | | | |

All of the animals in each group were positive for virus isolation from laryngeal swabs. The virus was isolated from almost all individuals between the third to the ninth day after challenge
(Table 7).

**Table 7**

| | Groups | Cat No. | Days after FIPV 79-1146 strain inoculation (days) | | | | | |
|---|---|---|---|---|---|---|---|---|
| | | | 0 | 3 | 6 | 9 | 12 | 15 |
| Virus isolation using fcwf-4 | Baculovirus recombinant N protein-immunized group | 177 | - | + | + | + | - | - |
| | | 242 | - | + | + | - | - | - |
| | | 245 | - | + | + | + | - | - |
| | | 247 | - | + | + | + | - | - |
| | Purified FIPV N Protein-immunized group | 175 | - | + | + | + | - | - |
| | | 180 | - | - | + | - | - | - |
| | | 243 | - | + | + | - | - | - |
| | | 252 | - | + | + | + | - | - |
| | SF-9 cell-derived antigen-immunized group | 178 | - | + | + | - | - | - |
| | | 181 | - | + | - | + | - | - |
| | | 244 | - | + | - | + | - | - |
| | | 249 | - | + | + | + | - | - |
| Detection of FCoV gene using RT-nPCR | Baculovirus recombinant N protein-immunized group | 177 | - | + | + | + | - | - |
| | | 242 | - | + | + | - | - | - |
| | | 245 | - | + | + | + | - | - |
| | | 247 | - | + | + | + | - | - |
| | Purified FIPV N protein-immunized group | 175 | - | + | + | + | - | - |
| | | 180 | - | + | + | + | + | + |
| | | 243 | - | + | + | - | - | - |
| | | 252 | - | + | + | + | - | - |
| | SF-9 cell-derived antigen-immunized group | 178 | - | + | + | - | + | - |
| | | 181 | - | + | - | + | - | - |
| | | 244 | - | + | - | + | - | - |
| | | 249 | - | + | + | + | + | - |

| | | | | | | | | |
|---|---|---|---|---|---|---|---|---|
| +: Virus was isolated, or FCoV gene was detected -: Virus was not isolated, or FCoV gene was detected | | | | | | | | |

### Virus isolation (RT-nested PCR):

The sensitivity of virus isolation from rectal swabs in challenge test (1) was low, and thus it was also carried out in challenge test (2). The rectal and laryngeal swabs were collected every three days after challenge, and used in the experiment. RT-PCR from rectal swabs yielded virtually the same results as those in challenge test (1), indicating that of the four animals, three animals in the recombinant N protein-immunized group, two animals in the purified N protein-immunized group, and three animals in the challenge control group were positive for one to two days (Table 6). According to RT-PCR from laryngeal swabs, all animals were positive in each group, and the virus was detected in almost all individuals between the third to the ninth day after challenge. However, on the 15th day after challenge, all animals except for Cat No. 180 became negative (Table 7).

### [10] Reactivity of feline coronavirus-infected cat sera to E. coli-expressed antigens

### Expression of recombinant proteins by E. coli:

For expression in *E. coli*, plasmid vector pGEX-2T (Pharmacia) was used to express fusion proteins with glutathione S-transferase (GST). Type I FIPV strain KU-2 gene was used for N protein expression. To express the entire N protein, the region from the initiation codon ATG to the stop codon TAA was inserted into a cloning site downstream of the GST coding region. The genes of the KU-2 strain as well as of the type II FIPV strain KU-1 were used for S protein expression. Within the S protein, a region encoding approximately 250 amino acids at the N terminal was cloned as for the N protein, excluding the signal peptide sequence, which is significantly varied depending on the strain. *E. coli* cells confirmed to be transfected were cultured at 37 °C for two hours. 1 mM of IPTG was added to the cells, and this was cultured for three hours to induce recombinant protein expression. This bacterial suspension was centrifuged to collect bacterial cells, washed with buffer, and then used in a Western blotting assay. The fusion protein of type I FIPV strain KU-2 N protein and GST was referred to as rIExN; that of the partial type I FIPV strain KU-2 S protein and GST was referred to as rIExS; and that of the partial type II FIPV strain KU-1 S protein and GST was referred to as rIIExS.

### Infected cat serum:

Five SPF cats were infected by inoculation with a culture supernatant of type I FIPV strains KU-2, Black, and UCD1, type II FIPV strain 79-1146, and type II FECV strain 79-1683, respectively. Blood was collected from each animal over time, and the serum with the highest antibody titer was used as the infected cat serum.

### Western blotting:

Sample buffer was added to the prepared antigens, which were then subjected to heat treatment, and applied to 11% gel SDS-PAGE. The gel was recovered and transferred onto a PVDF membrane. The membrane was washed, and then blocked by soaking overnight in BlockAce at 4°C. Infected sera from cats infected with each of the feline coronaviruses were used as primary antibody. The transferred membrane was soaked in primary antibody, reacted at 37°C for two hours, and then washed three times with 0.05% Tween20 PBS⁽⁻⁾. HRPO-conjugated anti-cat IgG + M + A antibody was used as the secondary antibody. It was soaked in antibody diluent supplemented with conjugated antibodies, reacted at 37°C for one hour, and then the membrane was washed three times with 0.05% Tween20 PBS⁽⁻⁾. The blotting membrane was stained by soaking in a substrate solution (0.02% DAB, 0.006% H₂O₂, and 0.05 M Tris-HCl (pH7. 6)), and then the reaction was stopped by washing with distilled water. The results are shown in Fig. 18.

The sera of cats infected with type I FIPV strains, KU-2 and Black, were responsive to the rIExS protein and rIExN protein, whereas the serum of UCD1 strain-infected cats was responsive to the rIExN protein, but not to the rIExS protein. The serum of cats infected with type II FIPV strain 79-1146 was responsive to the rIExS protein and rIExN protein. The serum of cats infected with type II FECV strain 79-1683 was responsive only to the rIExN protein. More specifically, recombinant N protein of the KU-2 strain indicated good reactivity towards all feline coronavirus-infected sera.

### Industrial Applicability

The present invention provides vaccines and methods for treating and/or preventing feline infectious peritonitis. The vaccines of this invention use proteins comprising amino acid sequences encoded by the polynucleotides of a) to e) as antigens. By using these proteins, vaccines with potential for preventive and/or therapeutic effects on a wide variety of strains can be achieved. Furthermore, since these proteins do not contain epitopes that enhance infection, the vaccines of this invention promise to be highly safe.

Feline infectious peritonitis is a serious infectious disease that often takes a lethal course after onset. Various vaccines have been produced to date, but their value has not been established. On the other hand, the vaccines of the present invention have excellent preventive effects compared to known vaccines, as well being very safe. Therefore, the vaccines of this invention are clearly useful in preventing and treating feline infectious peritonitis.

Furthermore, the present invention provides methods for diagnosing feline infectious peritonitis virus infections, in which the proteins comprising the amino acid sequences encoded by the polynucleotides of a) to e) are used as antigens, as well as reagents for such methods. The diagnostic methods and diagnostic reagents of this invention enable easy and rapid diagnosis of feline infectious peritonitis virus infections by a wide variety of viral strains. The methods for diagnosing feline infectious peritonitis virus infection are useful for screening to determine infection status. Investigation of infection status provides important information for FIP prevention.

## Claims

1. A subunit vaccine for treating and/or preventing feline infectious peritonitis, wherein said vaccine comprises a protein comprising an amino acid sequence encoded by a polynucleotide of any one of a) to e) as the active ingredient:
a) a polynucleotide comprising a coding region of the nucleotide sequence of SEQ ID NO: 1;
b) a polynucleotide comprising a nucleotide sequence that encodes the amino acid sequence of SEQ ID NO: 2;
c) a polynucleotide comprising a nucleotide sequence with 93% or more homology to a nucleotide sequence of a coding region of the nucleotide sequence of SEQ ID NO: 1;
d) a polynucleotide comprising a nucleotide sequence with 93% or more homology to the nucleotide sequence encoding the amino acid sequence of SEQ ID NO: 2; and
e) a polynucleotide encoding a continuous amino acid sequence comprising 45 or more amino acid residues, selected from an amino acid sequence encoded by the polynucleotide of a) or b).

2. A subunit vaccine for treating and/or preventing feline infectious peritonitis, wherein said vaccine comprises a polynucleotide of any one of a) to e) as the active ingredient:
a) a polynucleotide comprising a coding region of the nucleotide sequence of SEQ ID NO: 1;
b) a polynucleotide comprising a nucleotide sequence that encodes the amino add sequence of SEQ ID NO: 2;
c) a polynucleotide comprising a nucleotide sequence with 93% or more homology to a nucleotide sequence of a coding region of the nucleotide sequence of SEQ ID NO: 1;
d) a polynucleotide comprising a nucleotide sequence with 93% or more homology to the nucleotide sequence encoding the amino acid sequence of SEQ ID NO: 2; and
e) a polynucleotide encoding a continuous amino acid sequence comprising 45 or more amino acid residues, selected from an amino acid sequence encoded by the polynucleotide of a) or b).

3. The vaccine of claim 1 or 2, wherein the polynucleotide is the polynucleotide of a) or b).

4. Use of a protein as defined in claim 1 for the preparation of a medicament for treating and/or preventing feline infectious peritonitis.

5. A method of testing for feline infectious peritonitis virus infection, wherein said method comprises the steps of:
incubating cat serum with a protein comprising an amino acid sequence encoded by a polynucleotide of any one of a) to e):
a) a polynucleotide comprising a coding region of the nucleotide sequence of SEQ ID NO: 1;
b) a polynucleotide comprising a nucleotide sequence that encodes the amino acid sequence of SEQ ID NO: 2;
c) a polynucleotide comprising a nucleotide sequence with 93% or more homology to a nucleotide sequence of a coding region of the nucleotide sequence of SEQ ID NO: 1;
d) a polynucleotide comprising a nucleotide sequence with 93% or more homology to the nucleotide sequence encoding the amino acid sequence of SEQ ID NO: 2; and
e) a polynucleotide encoding a continuous amino acid sequence comprising 45 or more amino acid residues, selected from an amino acid sequence encoded by the polynucleotide of a) or b); and
detecting an antibody that binds to the protein.

6. A feline infectious peritonitis viral infection test reagent, comprising a protein that comprises an amino acid sequence encoded by the polynucleotide of any one of a) to e):
a) a polynucleotide comprising a coding region of the nucleotide sequence of SEQ ID NO; 1;
b) a polynucleotide comprising a nucleotide sequence that encodes the amino acid sequence of SEQ ID NO: 2;
c) a polynucleotide comprising a nucleotide sequence with 93% or more homology to a nucleotide sequence of a coding region of the nucleotide sequence of SEQ ID NO: 1;
d) a polynucleotide comprising a nucleotide sequence with 93% or more homology to the nucleotide sequence encoding the amino acid sequence of SEQ ID NO: 2; and
e) a polynucleotide encoding a continuous amino acid sequence comprising 45 or more amino acid residues, selected from an amino acid sequence encoded by the polynucleotide of a) or b).

## Patentansprüche

1. Untereinheiten-Impfstoff zum Behandeln und/oder Vorbeugen der Felinen Infektiösen Peritonitis, wobei der Impfstoff ein Protein, das eine Aminosäuresequenz umfasst, die durch ein Polynucleotid nach einem von a) bis e) kodiert wird, als wirksamen Bestandteil umfasst:
a) Polynucleotid, umfassend eine kodierende Region der Nucleotidsequenz der SEQ ID NO: 1;
b) Polynucleotid, umfassend eine Nucleotidsequenz, welche die Aminosäuresequenz der SEQ ID NO: 2 kodiert;
c) Polynucleotid, umfassend eine Nucleotidsequenz mit 93% oder mehr Homologie zu einer Nucleotidsequenz einer kodierenden Region der Nucleotidsequenz der SEQ ID NO: 1;
d) Polynucleotid, umfassend eine Nucleotidsequenz mit 93% oder mehr Homologie zu der Nucleotidsequenz, welche die Aminosäuresequenz der SEQ ID NO: 2 kodiert, und
e) Polynucleotid, das eine kontinuierliche Aminosäuresequenz kodiert, umfassend 45 oder mehr Aminosäurereste, ausgewählt aus einer Aminosäuresequenz, die durch das Polynucleotid nach a) oder b) kodiert wird.

2. Untereinheiten-Impfstoff zum Behandeln und/oder Vorbeugen der Felinen Infektiösen Peritonitis, wobei der Impfstoff ein Polynucleotid nach einem von a) bis e) als wirksamen Bestandteil umfasst:
a) Polynucleotid, umfassend eine kodierende Region der Nucleotidsequenz der SEQ ID NO: 1;
b) Polynucleotid, umfassend eine Nucleotidsequenz, welche die Aminosäuresequenz der SEQ ID NO: 2 kodiert;
c) Polynucleotid, umfassend eine Nucleotidsequenz mit 93% oder mehr Homologie zu einer Nucleotidsequenz einer kodierenden Region der Nucleotidsequenz der SEQ ID NO: 1;
d) Polynucleotid, umfassend eine Nucleotidsequenz mit 93% oder mehr Homologie zu der Nucleotidsequenz, welche die Aminosäuresequenz der SEQ ID NO: 2 kodiert, und
e) Polynucleotid, das eine kontinuierliche Aminosäuresequenz kodiert, umfassend 45 oder mehr Aminosäurereste, ausgewählt aus einer Aminosäuresequenz, die durch das Polynucleotid nach a) oder b) kodiert wird.

3. Impfstoff nach Anspruch 1 oder 2, wobei es sich bei dem Polynucleotid um das Polynucleotid nach a) oder b) handelt.

4. Verwendung eines wie in Anspruch 1 definierten Proteins zur Herstellung eines Medikaments zum Behandeln und/oder Vorbeugen der Felinen Infektiösen Peritonitis.

5. Verfahren zum Testen auf Infektion mit dem Felinen Infektiösen Peritonitis-Virus, wobei das Verfahren die Schritte umfasst:
Inkubieren von Katzenserum mit einem Protein, umfassend eine Aminosäuresequenz, die durch ein Polynucleotid nach einem von a) bis e) kodiert wird:
a) Polynucleotid, umfassend eine kodierende Region der Nucleotidsequenz der SEQ ID NO: 1;
b) Polynucleotid, umfassend eine Nucleotidsequenz, welche die Aminosäuresequenz der SEQ ID NO: 2 kodiert;
c) Polynucleotid, umfassend eine Nucleotidsequenz mit 93% oder mehr Homologie zu einer Nucleotidsequenz einer kodierenden Region der Nucleotidsequenz der SEQ ID NO: 1;
d) Polynucleotid, umfassend eine Nucleotidsequenz mit 93% oder mehr Homologie zu der Nucleotidsequenz, welche die Aminosäuresequenz der SEQ ID NO: 2 kodiert, und
e) Polynucleotid, das eine kontinuierliche Aminosäuresequenz kodiert, umfassend 45 oder mehr Aminosäurereste, ausgewählt aus einer Aminosäuresequenz, die durch das Polynucleotid nach a) oder b) kodiert wird, und
Nachweisen eines Antikörpers, der an das Protein bindet.

6. Testreagens für virale Infektion mit Feliner Infektiöser Peritonitis, umfassend ein Protein, das eine Aminosäuresequenz umfasst, die durch das Polynucleotid nach einem von a) bis e) kodiert wird:
a) Polynucleotid, umfassend eine kodierende Region der Nucleotidsequenz der SEQ ID NO: 1;
b) Polynucleotid, umfassend eine Nucleotidsequenz, welche die Aminosäuresequenz der SEQ ID NO: 2 kodiert;
c) Polynucleotid, umfassend eine Nucleotidsequenz mit 93% oder mehr Homologie zu einer Nucleotidsequenz einer kodierenden Region der Nucleotidsequenz der SEQ ID NO: 1;
d) Polynucleotid, umfassend eine Nucleotidsequenz mit 93% oder mehr Homologie zu der Nucleotidsequenz, welche die Aminosäuresequenz der SEQ ID NO: 2 kodiert, und
e) Polynucleotid, das eine kontinuierliche Aminosäuresequenz kodiert, umfassend 45 oder mehr Aminosäurereste, ausgewählt aus einer Aminosäuresequenz, die durch das Polynucleotid nach a) oder b) kodiert wird.

## Revendications

1. Vaccin à virus fractionné pour traiter et/ou prévenir la péritonite infectieuse féline, lequel vaccin comprend une protéine comprenant une séquence d'acides aminés codée par un polynucléotide de l'un quelconque parmi a) à e) à titre d'ingrédient actif :
a) un polynucléotide comprenant une région codante de la séquence de nucléotides de la SEQ ID NO : 1 ;
b) un polynucléotide comprenant une séquence de nucléotides qui code la séquence d'acides aminés de la SEQ ID NO : 2 ;
c) un polynucléotide comprenant une séquence de nucléotides ayant une homologie de 93 % ou plus vis-à-vis d'une séquence de nucléotides d'une région codante de la séquence de nucléotides de la SEQ ID NO : 1 ;
d) un polynucléotide comprenant une séquence de nucléotides ayant une homologie de 93 % ou plus vis-à-vis de la séquence de nucléotides codant la séquence d'acides aminés de la SEQ ID NO : 2 ; et
e) un polynucléotide codant une séquence d'acides aminés continue comprenant 45 résidus d'acides aminés ou plus, choisie parmi les séquences d'acides aminés codées par les polynucléotides de a) et b).

2. Vaccin à virus fractionné pour traiter et/ou prévenir la péritonite infectieuse féline, lequel vaccin comprend un polynucléotide de l'un quelconque parmi a) à e) à titre d'ingrédient actif :
a) un polynucléotide comprenant une région codante de la séquence de nucléotides de la SEQ ID NO : 1 ;
b) un polynucléotide comprenant une séquence de nucléotides qui code la séquence d'acides aminés de la SEQ ID NO : 2 ;
c) un polynucléotide comprenant une séquence de nucléotides ayant une homologie de 93 % ou plus vis-à-vis d'une séquence de nucléotides d'une région codante de la séquence de nucléotides de la SEQ ID NO : 1 ;
d) un polynucléotide comprenant une séquence de nucléotides ayant une homologie de 93 % ou plus vis-à-vis de la séquence de nucléotides codant la séquence d'acides aminés de la SEQ ID NO : 2 ; et
e) un polynucléotide codant une séquence d'acides aminés continue comprenant 45 résidus d'acides aminés ou plus, choisie parmi les séquences d'acides aminés codées par les polynucléotides de a) et b).

3. Vaccin selon la revendication 1 ou 2, dans lequel le polynucléotide est le polynucléotide de a) ou b) .

4. Utilisation d'une protéine telle que définie dans la revendication 1 pour la préparation d'un médicament destiné au traitement et/ou à la prévention de la péritonite infectieuse féline.

5. Procédé pour tester une infection par le virus de la péritonite infectieuse féline, lequel procédé comprend les étapes consistant à :
incuber du sérum de chat avec une protéine comprenant une séquence d'acides aminés codée par un polynucléotide de l'un quelconque parmi a) à e) :
a) un polynucléotide comprenant une région codante de la séquence de nucléotides de la SEQ ID NO : 1 ;
b) un polynucléotide comprenant une séquence de nucléotides qui code la séquence d'acides aminés de la SEQ ID NO : 2 ;
c) un polynucléotide comprenant une séquence de nucléotides ayant une homologie de 93 % ou plus vis-à-vis d'une séquence de nucléotides d'une région codante de la séquence de nucléotides de la SEQ ID NO : 1 ;
d) un polynucléotide comprenant une séquence de nucléotides ayant une homologie de 93 % ou plus vis-à-vis de la séquence de nucléotides codant la séquence d'acides aminés de la SEQ ID NO : 2 ; et
e) un polynucléotide codant une séquence d'acides aminés continue comprenant 45 résidus d'acides aminés ou plus, choisie parmi les séquences d'acides aminés codées par les polynucléotides de a) et b) ; et détecter un anticorps qui se lie à la protéine.

6. Réactif de test d'infection par le virus de la péritonite infectieuse féline, comprenant une protéine qui comprend une séquence d'acides aminés codée par le polynucléotide de l'un quelconque parmi a) à e) :
a) un polynucléotide comprenant une région codante de la séquence de nucléotides de la SEQ ID NO : 1 ;
b) un polynucléotide comprenant une séquence de nucléotides qui code la séquence d'acides aminés de la SEQ ID NO : 2 ;
c) un polynucléotide comprenant une séquence de nucléotides ayant une homologie de 93% ou plus vis-à-vis d'une séquence de nucléotides d'une région codante de la séquence de nucléotides de la SEQ ID NO : 1 ;
d) un polynucléotide comprenant une séquence de nucléotides ayant une homologie de 93 % ou plus vis-à-vis de la séquence de nucléotides codant la séquence d'acides aminés de la SEQ ID NO : 2 ; et
e) un polynucléotide codant une séquence d'acides aminés continue comprenant 45 résidus d'acides aminés ou plus, choisie parmi les séquences d'acides aminés codées par les polynucléotides de a) et b).


### SEQUENCE LISTING

<110> The Kitasato Institute
<120> FELINE INFECTIOUS PERITONITIS VACCINE
<130> EP33613Fzpau
<140> EP 03738684.4
   <141> 2005-01-28
<150> JP 2002-196290
   <151> 2002-07-04
<160> 8
<170> PatentIn Ver. 2.1
<210> 1
   <211> 1134
   <212> DNA
   <213> Feline infectious peritonitis virus
<400> 1
   atggccacac agggacaacg cgtcaactgg ggagatgaac cttccaaaag acgtgatcgt 60
   tctaactctc gtggtcggaa gaataataat atacctcttt cattcttcaa ccccaccacc 120
   ctcgaacaag gagctaaatt ttggtatgta tgtccgagag actttgttcc caagggaata 180
   ggtaataagg atcaacaaat tggttattgg aacagacagg cgcgctttcg cattgtcaag 240
   ggtcagcgta aggaactccc tgagagatgg tttttctatt tcttaggtac aggacctcat 300
   gctgatgcta aatttaaaga taagattgat ggagtcttct gggttgcaaa ggatggtgcc 360
   atgaacaagc caacatcact tggcactcgt ggaaccaaca atgaatccga accattgaga 420
   tttgatggta agataccacc acaattccag cttgaagtaa accgttctag gaataattca 480
   aggtctggtt ctcagtctag atctggctca agaaacaggt ctcaatccag gggaagacaa 540
   caatccaata accagaatac taatgttgag gatacaattg tagctgtgct tcagaaatta 600
   ggtgttactg acaagcaaag gtcacgttct aaatctagag accgtagtga ctctaaatct 660
   agagacacaa cacctaaaaa cgccaacaaa cacacctgga agaaaactgc aggtaagggt 720
   gatgtgacaa atttctttgg tgctagaagt gcttcggcta actttggtga tagtgatctc 780
   gttgccaatg gtaacgctgc caaatgctac cctcagatag ccgaatgcgt tccatcagta 840
   tctagcgtgc tcttcggtag tcaatggtcc gctgaagaag ctggagatca agtgaaagtc 900
   acacttactc acacctacta cctgccaaaa ggtgatgcca aaaccagtca attcctagaa 960
   cagattgacg cttacaagcg cccttcacaa gtagctaagg aacagaggaa accaaagcct 1020
   cgctctaagt ctgctgataa gaagcctgag gaattgtctg taactcttgt agaggcatac 1080
   acagatgtgt ttgatgacac acaggttgag atgattgatg aggttacgaa ctaa       1134
<210> 2
   <211> 377
   <212> PRT
   <213> Feline infectious peritonitis virus
<400> 2
   Met Ala Thr Gln Gly Gln Arg Val Asn Trp Gly Asp Glu Pro Ser Lys
   1               5                   10                  15
   Arg Arg Asp Arg Ser Asn Ser Arg Gly Arg Lys Asn Asn Asn Ile Pro
               20                  25                  30
   Leu Ser Phe Phe Asn Pro Thr Thr Leu Glu Gln Gly Ala Lys Phe Trp
           35                  40                  45
   Tyr Val Cys Pro Arg Asp Phe Val Pro Lys Gly Ile Gly Asn Lys Asp
       50                  55                  60
   Gln Gln Ile Gly Tyr Trp Asn Arg Gln Ala Arg Phe Arg Ile Val Lys
   65                  70                  75                  80
   Gly Gln Arg Lys Glu Leu Pro Glu Arg Trp Phe Phe Tyr Phe Leu Gly
                   85                  90                  95
   Thr Gly Pro His Ala Asp Ala Lys Phe Lys Asp Lys Ile Asp Gly Val
               100                 105                 110
   Phe Trp Val Ala Lys Asp Gly Ala Met Asn Lys Pro Thr Ser Leu Gly
           115                 120                 125
   Thr Arg Gly Thr Asn Asn Glu Ser Glu Pro Leu Arg Phe Asp Gly Lys
       130                 135                 140
   Ile Pro Pro Gln Phe Gln Leu Glu Val Asn Arg Ser Arg Asn Asn Ser
   145                 150                 155                 160
   Arg Ser Gly Ser Gln Ser Arg Ser Gly Ser Arg Asn Arg Ser Gln Ser
                   165                 170                 175
   Arg Gly Arg Gln Gln Ser Asn Asn Gln Asn Thr Asn Val Glu Asp Thr
               180                 185                 190
   Ile Val Ala Val Leu Gln Lys Leu Gly Val Thr Asp Lys Gln Arg Ser
           195                 200                 205
   Arg Ser Lys Ser Arg Asp Arg Ser Asp Ser Lys Ser Arg Asp Thr Thr
       210                 215                 220
   Pro Lys Asn Ala Asn Lys His Thr Trp Lys Lys Thr Ala Gly Lys Gly
   225                 230                 235                 240
   Asp Val Thr Asn Phe Phe Gly Ala Arg Ser Ala Ser Ala Asn Phe Gly
                   245                 250                 255
   Asp Ser Asp Leu Val Ala Asn Gly Asn Ala Ala Lys Cys Tyr Pro Gln
               260                 265                 270
   Ile Ala Glu Cys Val Pro Ser Val Ser Ser Val Leu Phe Gly Ser Gln
           275                 280                 285
   Trp Ser Ala Glu Glu Ala Gly Asp Gln Val Lys Val Thr Leu Thr His
       290                 295                 300
   Thr Tyr Tyr Leu Pro Lys Gly Asp Ala Lys Thr Ser Gln Phe Leu Glu
   305                 310                 315                 320
   Gln Ile Asp Ala Tyr Lys Arg Pro Ser Gln Val Ala Lys Glu Gln Arg
                   325                 330                 335
   Lys Pro Lys Pro Arg Ser Lys Ser Ala Asp Lys Lys Pro Glu Glu Leu
               340                 345                 350
   Ser Val Thr Leu Val Glu Ala Tyr Thr Asp Val Phe Asp Asp Thr Gln
           355                 360                 365
   Val Glu Met Ile Asp Glu Val Thr Asn
       370                 375
<210> 3
   <211> 30
   <212> DNA
   <213> Artificial Sequence
<220>
   <223> Description of Artificial Sequence:Artificially synthesized Sequence
<400> 3
   ggggaattca attaaaggca actactgcca 30
<210> 4
   <211> 41
   <212> DNA
   <213> Artificial Sequence
<220>
   <223> Description of Artificial Sequence:Artificially Synthesized sequence
<400> 4
   ctgtgaattc tgcaggatcc tttttttttt tttttttttt t 41
<210> 5
   <211> 20
   <212> DNA
   <213> Artificial sequence
<220>
   <223> Description of Artificial sequence:Artificially Synthesized sequence
<400> 5
   caactgggga gatgaacctt 20
<210> 6
   <211> 20
   <212> DNA
   <213> Artificial sequence
<220>
   <223> Description of Artificial Sequence:Artificially Synthesized sequence
<400> 6
   ggtagcattt ggcagcgtta 20
<210> 7
   <211> 22
   <212> DNA
   <213> Artificial Sequence
<220>
   <223> Description of Artificial Sequence:Artificially Synthesized Sequence
<400> 7
   attgatggag tcttctgggt tg 22
<210> 8
   <211> 23
   <212> DNA
   <213> Artificial Sequence
<220>
   <223> Description of Artificial Sequence:Artificially Synthesized sequence
<400> 8
   ttggcattct taggtgttgt gtc 23